# EUROPEAN PATENT SPECIFICATION

(11) **EP 4 210 666 B1**
(45) Date of publication and mention of the grant of the patent: **26.06.2024**
(21) Application number: 20768591.8
(22) Date of filing: 08.09.2020
(51) Int. Cl.: A61K 9/00, A61K 47/14, A61K 47/44, A61K 31/7024, A61K 36/185, A61K 36/258, A61K 36/28, A61K 36/45, A61K 36/53, A61K 36/54, A61K 36/752, A61K 36/87, A61K 36/9066, A61K 45/06

(54) **DUAL ACTING POLYMERS IN AN OSMOTIC FILM FOR TOPICAL APPLICATION TO TREAT INFLAMMATORY DISEASES AND CYTOKINE RELEASE SYNDROME**
DOPPELT WIRKENDE POLYMERE IN EINEM OSMOTISCHEN FILM ZUR TOPISCHEN ANWENDUNG ZUR BEHANDLUNG VON ENTZÜNDLICHEN ERKRANKUNGEN UND ZYTOKINFREISETZUNGSSYNDROM
POLYMÈRES À DOUBLE ACTION DANS UN FILM OSMOTIQUE POUR APPLICATION TOPIQUE POUR TRAITER DES MALADIES INFLAMMATOIRES ET LE SYNDROME DE LIBÉRATION DE CYTOKINES

(43) Date of publication of application: 19.07.2023
(73) Proprietor: VITROBIO SAS, 63500 Issoire (FR); Shrivastava, Léa, 63118 Cebazat (FR); Shrivastava, Rémi, 63110 Beaumont (FR)
(72) Inventor: SHRIVASTAVA, Ravi, 63118 CEBAZAT (FR); SHRIVASTAVA, Léa, 63118 CEBAZAT (FR); SHRIVASTAVA, Rémi, 63110 BEAUMONT (FR)
(74) Representative: Vidon Brevets & Stratégie
(86) International application number: PCT/EP2020/075117
(87) International publication number: WO 2022/053128

(56) References cited:
- WO-A1-2012/065651
- WO-A1-2014/194966
- JP-A- 2018 109 032

## Description

### FIELD OF THE INVENTION

The present invention relates generally to the fields of pharmacology and medicine. More specifically, the present invention relates to compositions for topical use in the prevention or the treatment of topical inflammatory disease and of the cytokine release syndrome in a subject in need thereof.

### BACKGROUND OF THE INVENTION

Almost all skin and mucosa injuries, whether of traumatic, allergic, toxic, viral, or bacterial origin, involve cellular damage that triggers a local inflammatory response by the immune system. This immune response is the very first protective and reparative response, leading to systemic reaction with the release of multiple disease related factors, cytokines and other proteins, that cause local inflammation, pain and oedema as common symptoms. To fight the pathogen, immune cells, particularly white blood cells, including B cells, T cells, natural killer cells, macrophages, dendritic cells, and monocytes gets activated and produce multiple disease specific cytokines at the site of infection or inflammation to supress the pathogen in the acute phase. If the infection continues, more and more pro-inflammatory cytokines are released to maintain inflammation as a defensive mechanism. If the disease is not healed and continues progressing, more and more immune cells continue activating which may stress and dysregulate the whole immune mechanism, suddenly releasing excessively high amount of multiple disease specific proteins and pro-inflammatory cytokines as a defensive mechanism. This phenomenon is known as Cytokine Release Syndrome (CRS), or Cytokine Storm (CS), that causes e.g. widespread severe inflammation (redness) leading to tissue damage, cellular fluid exudation, swelling, pain, heat, extravascular pressure, reduction in tissue perfusion, breach of cell membrane integrity, and the formation of intercellular gaps. The timing of symptom onset, CRS inducing cytokines, CRS severity and its consequences depends on the inducing agent, location of the disease, and the magnitude of immune cell activation. CRS leads to severe topical and systemic inflammation and generalised inflammation with variable symptoms depending upon the organs affected. In case of topical respiratory infections, systemic inflammation may particularly affect lungs causing leakage of pulmonary blood vessels, accumulation of fluid in lungs, widespread oedema, and respiratory distress, which may cause death if left untreated. The all-cause mortality attributed to CRS is estimated to be around 40%. IL-6 is considered as the main CRS inducing proinflammatory cytokine in lung-CRS but due to the supra-normal presence of other cytokines, the exact physiopathology of CRS is not yet established. When CRS does not affect vital organs, it may suddenly aggravate the pathology without being lethal.

This invention relates only to the topical diseases inducing local inflammation as the initial physio-pathological reaction. The initial topical lesion is usually caused by a pathogenic agent, an allergen, pollutants, or any substance interfering with cellular functions. The nasal and respiratory mucosa, oral cavity, throat surface, skin, vaginal and anal openings, gastrointestinal mucosa, and eye surface are the key organs where topical cellular damages are caused by an external aggression, but it can also be inflicted by internal noxious stimuli such as the herpes virus.

All the topical diseases that involve inflammation, cellular destruction and the breach of cellular integrity on any part of the body, can lead to CRS. Such topical diseases are, e.g., viral respiratory infections, nasal and ocular allergy, asthma, inflammatory respiratory diseases, ocular inflammatory disorders, psoriasis, eczema, dermatitis, haemorrhoids, chronic wounds, diabetic and gastric ulcers.

CRS is commonly observed in viral diseases involving skin or mucosa, such as influenza and corona viruses related diseases, which cause cellular destruction and chronic inflammation.

Many viruses have an external capsid which contains specific glycoproteins (Gps) on their surface. Example of viral Gps include H (hemagglutinin) and N (neuraminidase) Gps on influenza virus; Gps C (gC) and B (gB) on herpes virus; spike S (S1 and S2) Gps on COVID-19, SARS, & MERS of corona viruses. Infection begins when the viral glycoprotein attaches to its complementary host cell receptor to infect and to enter the cell for multiplication. Many viruses cannot break the host cell wall and require the help of topically available proteolytic enzymes (proteins) to enter the cell. Virus kills the cells and millions of free virus particles are liberated on the infected surface which start propagating in surrounding tissues and infecting new cells. Cell death leads to the formation of gaps in the natural cellular barrier allowing systemic entry of other pathogens.

In case of nasal or throat viral infections, the virus progressively infects the upper and lower respiratory tract mucosa and reaches up to the lungs. If the virus spreading is not stopped rapidly, it leads to extensive cellular damage and inflammation of the throat and respiratory mucosa.

Nasal and ocular allergy, asthma and inflammatory respiratory diseases are mainly caused by viral infections, allergens, or pollutants. The causative agents enter the nasal, oral or ocular mucosa from environmental exposure and trigger local irritation, inflammation and cellular damage producing gaps in the mucous membrane through which inflammatory proteins can enter systemic circulation. The mucous membranes of these organs contain sensory TRP receptors (Transit Receptor Potential channels), the stimulation of which trigger neurogenic inflammatory cascade due to the production of TSLP (Thymic Stromal Lymphoprotein). Inflammatory and allergen cascades activate dendritic cells and TH2 lymphocytes leading to the release of inflammatory cytokines (ex. IL-4, IL-6, IL-23, IL-33, TNF- α), followed by the activation of B-lymphocytes, and liberation of IgE antibodies. IgE binding on the surface of mast cells trigger release of histamine, leukotrienes, prostaglandins and other proteins which start accumulating topically on the damaged surface. Although the basic physiopathology of inflammation remains identical, the location, type and the concentration of these proteins may vary from disease to disease (disease specific proteins) requiring different approaches to treat each disease.

If topical inflammation is left untreated, inflammation destroys mucosa cells, creating intercellular gaps due to the loss of intercellular connective tissue. These cellular gaps allow direct systemic entry of topical proteins, cytokines, allergens and pollutants, which maintain inflammatory and immune cascades leading to chronic inflammation, extensive cellular destruction, chronic allergy, respiratory distress, asthma, then increasing the risk of triggering CRS. If the pathogen exposure is not blocked, the sensitization of TRP channels and other pro-inflammatory receptor are not stopped, the concentration of surface cytokines and undesired proteins such as histamine, IgE, and TSLP is not reduced, and if the cellular gaps are not healed, the disease becomes chronic and CRS is more likely to occur.

It has also been shown that some drugs can provoke CRS, such as, to name a few, rituximab, CD19 CAR-T cell Tisagenlecleucel, TGN1412 or Theralizumab and other immune system modulating biotherapeutics such as Muromonab-CD3 and Alemtuzumab. The mechanism remains identical except that these drugs may modulate and dysregulate the immune system. Psoriasis, eczema and dermatitis are immune diseases, in which a deregulation of growth factors leads to excessive and uncontrolled cell growth, skin drying and sloughing. Poor skin cell adherence leads to the entry of bacterial contaminants in the lesion which start destroying cells. Cellular destruction leads to the release of multiple pro-inflammatory cytokines on the surface and the disease becomes chronic.

Chronic wounds such as bedsores and diabetic ulcers are extremely difficult to heal as, in addition to containing dead cells, cell debris, and multiple proteolytic enzymes which impede cell growth and wound repair, those lesions remain open to the external environment and often get contaminated. This is the reason why most chronic wounds such as bedsores, diabetic ulcers, or venous leg ulcers, never heal, and nearly 40% patients die before they experience wound resolution. Would healing requires cell growth which in turn requires a clean, chemical free, inflammation free, and hydrated environment. To clean the injury, the human body naturally produces more than 27 different Matrix Metalloproteinases (MMPs). These proteolytic enzymes are directed to the lysis of protein debris into smaller particles, which can be removed through wound exudation. But it is now well established that some of these MMPs (MMP 2, 3, 8, 9, and 13) destroy not only the protein debris but also the cellular matrix building proteins, such as the collagen, elastin, laminin, and hyaluronic acid. In the absence of this cellular matrix, cells cannot attach, cannot grow, and the wound cannot heal.

Haemorrhoids present dilated and inflamed blood vessels with specific hemorrhoidal proinflammatory cytokines on the surface.

All of the above-mentioned examples of topical diseases on the skin, nasal, oral, gastric, and respiratory mucous or on the eye surface show that they always involve cellular destruction and the breach of cellular integrity. Focal and topical cellular destruction leads to bacterial growth, triggering of inflammatory reaction with the release of disease related specific pro-inflammatory cytokines (e.g. interleukins) and other proteins (e.g. histamine, MMPs, IgE) on the damaged surface. In addition, according to the origin of the disease, the lesion may also contain the pathogens such as virus particles, allergens, pollutants, heavy metal particles such as arsenic, mercury, cadmium, and lead.

Unfortunately, since CRS has a multi-factorial origin, there is currently no treatment efficient in preventing or treating CRS. An ideal treatment must be multi-target to prevent the occurrence of CRS as it should: i) clean the infected surface by trapping and/or removing the noxious compounds (bacterial contaminants, pathogens and all other contaminants), ii) protect the infected surface against a new infection, iii) provide a favourable clean environment for cell growth and to fill-up the intercellular gaps. But it must also iv) stop or reduce the detrimental cytokine release triggering cycle and v) supress inflammation to reduce irritation, pain, itching and further cellular damage. If any of these factors are not properly addressed, recovery will be delayed and, in the worst case, the cytokine storm may happen and lead to death.

For example, pulmonary cytokine syndrome due to coronavirus infection is caused by the sudden release of large quantities of cytokine, mainly IL-6, which may cause severe lung inflammation and oedema. Death may occur due to respiratory failure. Other CRSs which do not affect lungs, are not evident to detect as they aggravate the inflammation and worsen the disease without causing heart or lung damage. To treat coronavirus induced pulmonary inflammation, specific anti-IL-6 antibodies (tocilizumab) were found poorly effective and are still not approved for preventing from CRS. Other treatments such as high dose corticosteroids, absorbing cytokines with extracorporeal hemofiltration, antibody rich plasma transfusion, or chloroquine treatments were found to be poorly effective.

Other anti-CRS symptomatic treatments are mono-target and poorly effective since they are solely directed against inflammation (anti-inflammatory drugs), or microbial contaminants (antiseptics and antibiotics), intracellular viruses (Tamiflu which acts intracellularly as topical antivirals are not yet discovered), pain relief (anaesthetics or analgesics), or the blockage of one specific cytokine.

For centuries, plant extracts rich in tannins have been used topically as wound healing helping agents, reducing contaminants, or decreasing inflammation and pain but results are not satisfactory because probably the plant tannins bind to only a few non-selective proteins or cytokines and probably they also inhibit the good proteins which are essential for healing.

Among the treatments dedicated to the cleaning of injured surface, sea water or saltwater gargles is still considered one of the best remedies against throat infection or inflammation. These osmotically active solutions form a hypertonic film over the throat mucosa and the resulting outward exudation of hypotonic liquid helps reduce the contaminant load on the throat or wound surfaces. Unfortunately, this hypertonic solution film gets diluted within a few minutes by the outflowing hypotonic liquid, limiting the efficacy of that treatment. Furthermore, the NaCl concentration (maximum 3.4%) has low osmotic power to exert a strong osmotic effect. This concentration cannot be increased because of the resulting strong irritation, cellular damage, mucosa burning, and chemical induced cellular cytotoxicity.

The document WO 2014/194966 discloses the use of hypertonic osmotically active compositions for topical application to clean superficial injuries through osmotic liquid flow. These compositions comprise glycerol and plant tannins which are able to bind the glycerol, in order to obtain a composition, also called "filmogen glycerol", with increased retention time on a living biological surface. Such a filmogen glycerol composition can be used as an effective topical agent to clean an injured surface. The osmotic pressure exerted by the glycerol film, attracts hypotonic liquid from the inner parts of the tissue thereby detaching and draining the surface contaminants. Minimizing damaged surface contact with the incoming pathogens and reducing the concentration of contaminants, stimulates cell growth and the formation of an intact cellular barrier. However, the tannins in filmogen glycerol are directed to bind only with glycerol and not with any other disease related specific proteins or macromolecules. It has no or only little effect on the reduction of very small molecules such as the cytokines, other disease related proteins or cytotoxic macromolecules which play a key role in maintaining inflammation and in delaying the healing process. As they cannot be eliminated totally through osmotic liquid flow, noxious molecules such as the virus particles, inflammatory cytokines, histamine, IgE, growth factors and other small proteins, continue to exert their detrimental effects. The inventors observed that among millions of virus particles or cytokines liberated on the damaged surface, even if 99% particles are eliminated through the osmotic flow, the remaining 1% is sufficient to infect new cells or to maintain inflammatory cascade. Although the osmotically active glycerol film removes a great amount of free-floating surface molecules, it fades with time, the osmotic pressure exerted decreases along with the cleaning efficiency over long periods. The remaining small noxious molecules continue to maintain inflammatory, allergenic and immune cascades which can trigger CRS.

WO 2012/065651 describes compositions for topical application, said compositions comprising a synergistic association of plant extracts containing specific tannins which have been selected to neutralize cell growth stimulating cytokines. The extracts can be mixed with a carrier. There is an important need to find a multi-target mechanism which, in addition to protecting and cleaning the injured surface, can simultaneously neutralize disease specific pro-inflammatory cytokines, inflammation triggering TRP receptors, proteins, pathogens and/or other noxious molecules, to supress inflammation and to minimize the risk of CRS and its consequences.

Such compositions should be totally safe, non-cytotoxic, fast acting, topically applicable to avoid systemic absorption.

### SUMMARY OF THE INVENTION

The scope of this invention is defined by the claims. Any references in the description to methods of treatment refer to the pharmaceutical compositions for use in a method for treatment of the human (or animal) body by therapy. The present invention provides a composition for use in preventing or treating topical inflammatory disease and the cytokine release syndrome (CRS). The invention relates to compositions, which act on multiple targets involved in the triggering of CRS, and which may be used in human beings or animal subjects.

The present invention relates to a composition comprising at least one dual acting polymer bound to glycerol and able to bind to at least one pro-inflammatory compound, for topical use in the prevention or the treatment of topical inflammatory disease and of the cytokine release syndrome, in a subject in need thereof. According to the claimed invention, the composition is for topical use in the prevention or the treatment of the cytokine release syndrome.

In an embodiment, the at least one pro-inflammatory compound is selected from the group consisting of matrix metalloproteases, histamines, cytokines, cellular receptors, metal ions, immunoglobulins or viral glycoproteins.

In an embodiment, the topical inflammatory disease is selected from the group consisting of viral infections, rhinosinusitis, wounds and ulcers, psoriasis, eczema, dermatitis, allergy, asthma, pollution induced respiratory diseases, pollution induced topical damage, gastro-intestinal ulcers, haemorrhoids, genital infections, ocular allergy, conjunctivitis, and ocular inflammation. In a preferred embodiment, the total amount of the at least one dual acting polymer is ranging from 0.01% to 5% by weight of total weight of said composition, preferably from 0.01% to 3.5% by weight of total weight of said composition.

In another embodiment, the composition is further comprising at least one ingredient selected from the group consisting of honey, propolis extract, vegetable gum such as xanthan gum and/or acacia gum, and essential oils.

In another embodiment, the composition is further comprising at least one medication, such as an analgesic, antibiotic, anti-inflammatory drug, antihistamine, vasodilator, bronchodilator, antioedematous drug, specific topical receptor binding compound or an essential oil.

In an embodiment, the composition is topically applied on a damaged and/or inflammatory biological surface such as the skin, eye mucosa, conjunctiva, cornea, oral, nasal, gastrointestinal, respiratory, or genital surfaces.

In an embodiment, the composition is administered as a liquid, inhaler, liquid bandage, solution, gel, cream, paste, or ointment, presented in sprays, tubes, ampoules, liquid embedded cotton or polymeric bandages, granules, powder or soft-gel capsules.

In an embodiment, the at least one dual acting polymer is natural, semi-synthetic and/or synthetic.

In another embodiment, the at least one dual acting polymer is a tannin obtained from a plant or parts of the plant.

In another embodiment, the ingredient and/or the medication is captured into the polymeric linkages of said composition, for sustained release of the ingredient and/or of the medication, to further enhance therapeutic properties of the composition.

Also described herein is a method for obtaining a composition comprising at least one dual acting polymer bound to glycerol into which an ingredient and/or a medication is captured for sustained release, to further enhance therapeutic properties of the composition. The method of the invention comprises the steps of:
- mixing glycerol with an ingredient and/or a medication, and then
- adding at least one dual acting polymer to form a glycerol-dual acting polymer mesh around the ingredient and/or the medication.

### BRIEF DESCRIPTION OF THE FIGURES

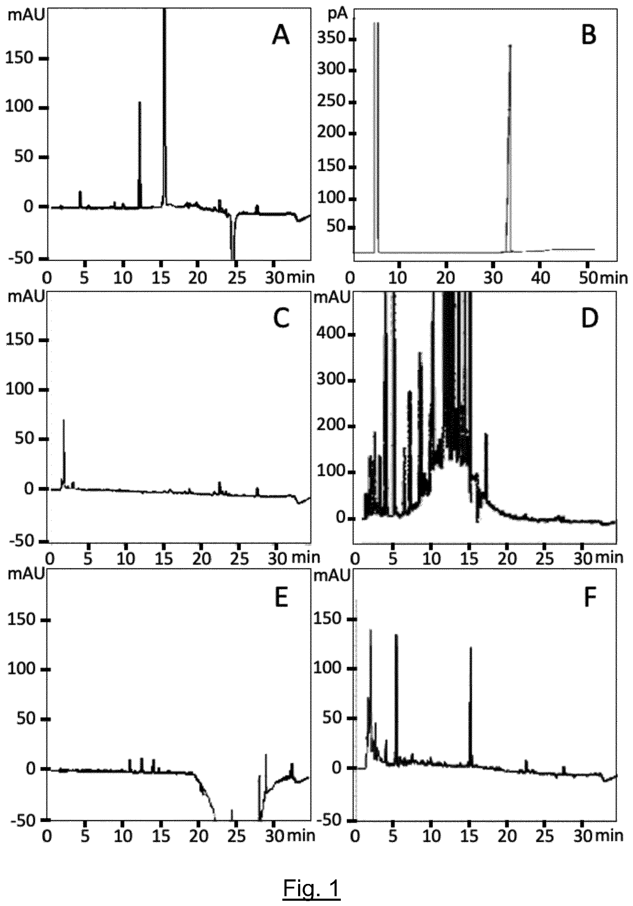

**Figure 1:** (A) Glycerol containing 4 dual acting polymers: *P. ginseng* + *C. sinensis* + *C. longa* + *U. dioica;* (B) GCMS profile of glycerol. Glycerol is detected as a single peak between 31-32 minutes with a pA of 340; (C) *P. ginseng* alone; (D) *C*. *sinensis* alone; (E) *C*. *longa* alone; (F) *U. dioica* alone.

### DETAILED DESCRIPTION OF THE INVENTION

Within the context of the invention, the term "inflammatory topical diseases" or "topical inflammatory diseases" designates all inflammatory diseases where pathological symptoms are manifested on the surface of an organ or the body such as skin, oral cavity, nasal cavity involving upper and lower respiratory tract, throat and/or eye surfaces, genital cavity, anal opening, as well as gastro-intestinal (GI) mucosa.

Within the context of the invention, the terms "multifactorial disease" means that there are multiple factors acting together to cause the occurrence of the disease. In all the topical diseases when inflammation becomes chronic, over activation of immune reaction floods the lesion with multiple pro and anti-inflammatory cytokines and other proteins, which may cause widespread tissue destruction. For example, in topical allergic reaction, there is cellular damage, presence of multiple inflammatory cytokines, TSLP, histamine, IgE antibodies and allergens on the allergic surface, which help continue the allergic reaction. Examples of topical multifactorial diseases involving cytokines are e.g. viral and bacterial throat infection, rhinosinusitis, cough, allergic and pollution induced rhinitis, asthma, genital infections, chronic skin and mucosa ulcers and wounds, topical vascular pathologies such as the haemorrhoids, and ocular inflammatory disease. Disease related cytokines and their concentration may vary according to the type of infection, location of the injury, type of cells involved, and the extent of tissue damage. Therefore, the topical inflammatory diseases according to the invention can be broadly divided into five categories: (1) chronic skin and mucosa wounds (2) nasal, oral, genital and skin inflammatory diseases, (3) respiratory diseases, (4) viral diseases, (5) ocular diseases.

In an embodiment, the topical inflammatory disease is selected from the group consisting of viral infections, rhinosinusitis, wounds and ulcers, psoriasis, eczema, dermatitis, allergy, asthma, pollution induced respiratory diseases, pollution induced topical damage, gastro-intestinal ulcers, haemorrhoids, genital infections, ocular allergy, conjunctivitis, and ocular inflammation. Within the context of the invention, the prevention or the treatment of CRS means the prevention or the treatment of the multiple factors involved in the topical inflammatory diseases. All these CRS triggering factors must be either reduced or blocked simultaneously to minimize inflammatory threshold which triggers CRS. The key factors therefore lie in stopping contact with the cause, re-establishing natural mucosa barrier functions by cleaning the inflamed surface to stimulate cell growth, and by minimizing the concentration of pro-inflammatory triggers, i.e. specific cytokines, other proteins, cell surface receptors, or macromolecules (CPRMs) simultaneously. In the context of the invention, "pro-inflammatory compound" and "CPRM" are both used to designate the proteins, cell surface receptors, macromolecules, allergens and pollutants such as metal ions and other compounds that are related to the topical inflammatory reaction.

In the context of the invention, the terms "neutralization" and "blocking" designate the loss of activity of the target molecule either because it is expelled from the site or bound to other molecules in such a way that it cannot exert its function.

An object of the present invention relates to compositions comprising at least one dual acting polymer bound to glycerol on one hand and able to bind with one or more CPRM, for topical use to minimize topical inflammation and the chances of triggering of CRS, in a subject in need thereof. Dual acting polymers are able to bind with glycerol but also to one or more pro-inflammatory compounds, receptors, or noxious molecules.

Glycerol or glycerine is a high boiling, viscous, colourless, odourless hydroscopic, and sweet solution. It is highly osmotic and has the capacity to attract hypotonic liquid through a semipermeable membrane. It is a trihydroxy sugar alcohol molecule with three basic backbones of three carbon atoms, each of them covalently bonded to a hydroxyl group.

The presence of multiple free hydroxyl groups and carbon atoms makes it an organic polyol compound with the IUPAC name of 1, 2, 3 - propanetriol. The three hydroxyl groups of glycerol allow reactions with many organic acids to form esters and with the polymeric substances to form macromolecules.

Glycerol is not very irritant but high concentrations may irritate sensitive tissues. Therefore, the concentration of glycerol in the compositions can be selected on the basis of the necessity of hydrating and cleaning properties required to treat a particular topical pathology and the sensitivity of the biological tissue onto which the composition will be applied. For example, pharyngitis is often accompanied by severe bacterial infection where microorganisms are strongly adhered to the throat surface, requiring a strong osmotic force to detach and drain the contaminants. Throat surface being less sensitive to irritation, the concentration of glycerol in the filmogen glycerol was high (above 50%). To treat rhinosinusitis, nasal mucosa being the most sensitive organ in the body, the concentration of glycerol was kept low (below 30%). Compositions containing higher concentration of glycerol and glycerol binding tannins or polymers with low excipients (water), form a thin film resistant to mechanical forces compared to low concentration of glycerol. Fortopical injuries requiring strong cleaning activity, the glycerol concentration was high (up to 99%) while low concentrations were used for sensitive damaged surfaces and those requiring lower osmotic cleaning.

According to the invention, the compositions for topical use also comprise dual acting polymers. All polymers, whether natural or synthetic, have a strong capacity to bind with specific proteins (such as cytokines, histamine, IgE) but also with various other organic compounds including macromolecules, allergens, heavy metal particles, metallic ions, amino acids and alkaloids. The polymer-protein or polymer-macromolecule binding is specific as all the polymers cannot bind with all the molecules, but they can bind with more than one molecule at a time due to their large size. The "dual acting polymers" of the invention are polymers that have the capacity to bind with glycerol molecules and simultaneously, in addition, with one or more specific CPRMs, i.e. specific cytokines such as IL-6, other proteins such as IgE, cell surface receptor such as TRP receptor, or macromolecules such as As, Pb, Hg. This particular feature is essential because if a polymer or an association of polymers capable of blocking specific CPRMs or metal ions is applied directly on an injury, it gets rapidly expelled through osmotic liquid flow as it is not adhered to the glycerol molecules and it is not a part of filmogen glycerol. Therefore, only those natural, semi-synthetic or synthetic polymers having capacity to bind not only with glycerol molecules but also with one or more disease related pro-inflammatory CPRMs, can exert dual activity. If only CPRMs binding polymers are applied on the damaged surface, it may block a few proteins but protective and lesion cleaning effect of glycerol will be absent. In this case, cells will not grow, pathogens will continue entering in the body, and inflammation will persist.

A few examples of preferred pro-inflammatory compound (CPRMs) are disclosed in Table 1 below.

**Table 1**

| Condition | Pro-inflammatory compounds (CPRMs) |
|---|---|
| Chronic skin and mucosa injuries | Matrix metalloproteinases (MMP)-1, MMP-2, MMP-3, MMP-8, MMP-9, MMP-13, TNF-α, and IL-1β |
| Inflammatory diseases | Interleukin (IL)-1, TNF-α, IFN-y, IL-4, IL-5, IL-6, IL-10, IL-13 |
| Respiratory, allergen or pollution related diseases | Histamine, IgE, TRP receptors, TSLP, (IL)-3, IL-4, IL-5, IL-6, IL-9, IL-13, IL-23, IL-25, IL-33 and metal ions such as As, Cd, Pb, and Hg |
| Viral diseases | Viral Glycoproteins, virus entry enhancing proteases |
| Ocular diseases | TSLP proteins, TRP receptors, histamine, IgE, metal ions |

According to a preferred embodiment, the dual acting polymers of the invention are able to bind to at least one compound selected from the group consisting of matrix metalloproteases, histamines, cytokines, TRP receptors, metal ions, immunoglobulins and viral glycoproteins.

In an embodiment, the dual acting polymer(s) is able to bind to at least one cytokine selected from the group consisting of chemokines, interferons, interleukins, lymphokines, tumour necrosis factors, thymic stromal lymphopoietin (TSLP) and growth factors.

Pollutants are substances introduced into the environment that have undesired effects, or adversely affect the usefulness of a resource, such as vehicle exhaust fumes containing cytotoxic molecules or macromolecules such as the heavy metal particles (As, Cd, Hg, and Pb). Hence, in an embodiment, the dual acting polymer(s) is able to bind to at least one metal ion selected from arsenic, cadmium, lead and/or mercury.

Matrix metalloproteases (MMPs), also known as matrix metalloproteinases or matrixins, are proteolytic enzymes which are involved in cleaning the injury through proteolysis, in modulating cell growth but also in degrading all kinds of extracellular matrix proteins. They are also involved in processing several bioactive molecules, interacting with cell surface receptors, releasing apoptotic ligands, chemokine/cytokine inactivation, cell migration (adhesion/dispersion), differentiation, angiogenesis, apoptosis, and in host defence.

In an embodiment, the dual acting polymer(s) is able to bind to at least one matrix metalloproteases (MMP) selected from MMP-1, MMP-2, MMP-3, MMP-8, MMP-9, MMP-13.

In another embodiment, the dual acting polymer(s) is able to bind to at least one cell surface receptor, preferably a receptor of the transient receptor potential (TRP) channels, such as TSLP releasing TRP receptors.

The concentration of dual acting polymers, whether natural, synthetic, or semi-synthetic, in the composition, should be as low as possible and should not exceed those levels which interfere with the formation of the film and their CPRMs binding potential. Hence, in an embodiment, the total content of dual acting polymer(s) varies from about 0.01% to about 5% by weight of total weight of the composition, preferably from about 0.01% to about 3.5% by weight of total weight of the composition. In a particular embodiment, the total content of dual acting polymer(s) is less than 2.0% by weight of total weight of the composition. Such concentrations allow to conserve the osmotic activity of filmogen glycerol. Moreover, the concentration of polymers used in compositions should not be cytotoxic in the concentrations used.

The quality, quantity and association of dual acting polymers can be modified according to the site of topical application and the state of the injured surface.

Examples of synthetic polymers are polythene, synthetic rubber, polyvinyl chloride (PVC), Poly[imino(1,6-dioxohexamethylene) iminohexamethylene] (trade mark Nylon-66), polytetrafluoroethylene (trade mark Teflon), acrylic fiber (e.g. trade mark Orlon), Carboxymethylcellulose under sodium salt (CMCNa, also called Carmellose), Poly(vinyl alcohol) (PVOH), Polyacrylamide (PCM), Sodium polyacrylate (SPA), Polyethylene glycol (PEG), Pluronic F-127 (PL127), Klucel- Hydroxypropyl cellulose (HPC) and/or Solagum (association of acacia gum & xanthan gum). As polymers are very big molecules and cannot be absorbed in the body, even synthetic polymers can be used for topical application.

Natural polymers such as tannins are polymeric phenolic compounds found in plant materials having molar masses ranging from 300 Da to 3000 Da, and even up to 30,000 Da. In an embodiment, the natural dual acting polymers are obtained from any plant or part of the plant which is rich in tannins. The extraction methods of a natural polymer from a plant or a part of a plant are well known in the art, as for example extractions by maceration, Soxhlet, or by percolation techniques, as given in Reference Dang Xuan Cuong *et al.* (2019).

The synthetic or semi-synthetic dual acting polymers are prepared chemically or modified chemically from the natural polymers.

The synthetic, semi-synthetic or natural dual acting polymers of the invention can be used in combination according to specific disease related pro-inflammatory CPRMs that have to be blocked.

To obtain the dual acting polymers according to the invention, a non-cytotoxic polymer is first selected and then its ability to bind to glycerol and to the one or more pro-inflammatory compound (CPRM) is verified. It can be first checked if the polymer is able to bind to glycerol and, if the polymer binds with glycerol, it is further checked whether it binds to one or more disease specific CPRMs or vice versa. There are several methods in the art for verifying that the polymer is able to bind to glycerol and to one or more CPRM.

An example of a method for checking the ability of the polymer to bind to glycerol is to perform a first GC/HPLC profile of the polymer alone and to compare it with a second GC/HPLC profile of the combination of the polymer with glycerol. GC or HPLC profiles can be obtained with methods that are well known in the art. If the first profile is different from second profile, it means that the polymer has bound to the glycerol molecule. The minimum concentration of polymer capable to bind with 100% glycerol molecules is selected for CPRM binding evaluations.

The technique for evaluating disease specific CPRM binding is selected according to the type of CPRM. For example, ELISA immunoassay is well adapted to evaluate polymer binding with cytokines or a protein receptor. The metal ion binding can be evaluated by incubating the polymer(s) with the 50% cytotoxic concentration of one or more metal ion such as arsenic or cadmium followed by comparing the difference of cytotoxicity, before and after incubation, in specific cell cultures . If the toxicity is reduced, it can be assumed that the CPRM is bound to the polymer. To evaluate virus neutralization by a polymer(s), the polymer(s) can be incubated with a known cytotoxic concentration of a specific virus (e.g. influenza, herpes, corona viruses) followed by exposing the polymer-virus association to virus sensitive cells in vitro. Reduction in cytotoxicity of polymer incubated mixture compared to non-polymer-incubated virus controls, show virus neutralization. The concentration of polymer(s) required to neutralize 100% virus can be quantified. If in vitro or immunological experiments are not possible (e.g. for volatile metals like Hg), the CPRM can be administered orally or topically in sensitive animal models (e.g. rat, mice, Guinea pig) and the difference of toxicity with polymer fed and non-polymer fed parameters can be quantified. The reduction of CPRM-polymer(s) related toxicity compared to CPRM alone should indicate CPRM neutralization due to polymer binding. The efficacy of CPRM neutralizing formulations can be verified in animal models or in human clinical trials.

Hence, depending on the inflammatory topical disease to treat, and consequently on the CPRM(s) involved, it is possible to select from the thousands of existing natural, semi-synthetic or synthetic polymers, those that are dual acting and that are suitable according to the present invention.

Thickening and jellifying agents can be further incorporated in the compositions of the invention to increase the thickness and the absorbent capacity of the film, in particular in topical pathologies where environmental contaminants (ex. allergens - pollen, metal ions, pollution) continue attacking the biological surface (ex. nasal mucosa). The compositions may also contain preservatives. In an embodiment, the compositions of the invention further comprise at least one thickening / jellifying ingredient selected from the group consisting of honey, propolis extract, vegetable gum such as xanthan gum and/or acacia gum, and essential oils or a stabilizer selected in the group of the preservatives, such as potassium sorbate, sodium benzoate and citric acid.

To create an environment for cell growth, it is also important to use only cell friendly concentrations of the substances which are not cytotoxic.

In an embodiment, the compositions of the invention further comprise at least one pharmaceutically acceptable excipient.

In an embodiment, the compositions of the invention further comprise at least one medication, such as an analgesic, antibiotic, anti-inflammatory drug, antihistamine, antiviral, vasodilator, bronchodilator, antioedematous drug, specific topical receptor binding polymer and essential oils.

Examples of suitable analgesic are salicylic acid, paracetamol, morphine, CGRP or cox-2 inhibitors, acetaminophen, NSAIDs, or triptans.

Examples of suitable antibiotic are beta-lactams, aminoglycosides, tetracyclines, glycylcyclines, macrolides, azalides, ketolides, synergistins, lincosanides, fluoroquinolones, phenicols, rifamycins, sulfamides, trimethoprim, glycopeptides, oxazolidinones, nitromidazoles and lipopeptides.

Examples of suitable anti-inflammatory drug are salicylate and salts thereof, celecoxib, diclofenac, diflunisal, etodolac, fenoprofen, flurbiprofen, ibuprofen, indomethacin, ketoprofen, meclofenamate, mefenamic acid, meloxicam, nabumetone, naproxen, oxaprozin, piroxicam, rofecoxib salsalate, sulindac, tolmetin, valdecoxib, prednisone, methylprednisolone, prednisolone, aldosterone, cortisol, cortisone, hydrocortisone, corticosterone, tixocortol, ciclesonide, prednicarbate triamcinolone acetonide, triamcinolone alcohol, mometasone, amcinonide, budesonide, desonide, fluocinonide, fluocinolone acetonide, halcinonide, hydrocortisone-17-valerate, halometasone, alclometasone, betamethasone, prednicarbate, clobetasone-17-butyrate, clobetasol-17-propionate, fluocortolone, fluocortolone, fluprednidene acetate, dexamethasone.

Examples of suitable antihistamine drug are cetirizine, brompheniramine, chlorpheniramine, clemastine, fexofenadine, loratadine.

Examples of suitable vasodilator are benazepril, captopril, enalapril, lisinopril, moexipril, quinapril, perindopril.

Examples of suitable bronchodilator are beta-2 agonists, anticholinergics, and theophylline. Examples of suitable antioedematous drug are diuretics such as furosemide and topical anti-inflammatory drugs.

Examples of suitable specific topical receptor binding compound drug are rosiglitazone, capsaicin, diphenyleneiodonium chloride, linopirdine and similar agonists and antagonists. Examples of suitable essential oil are *Mentha piperita* essential oil, *Eucalyptus globulus* essential oil, *Rosmarinus officinalis* essential oil, *Thymussatureioides* essential oil, *Linum usitatissimum* oil, *Citrus limonum* essential oil, and combination thereof.

The compositions of the invention can be obtained by mixing the glycerol with the at least one dual acting polymer. In an embodiment, to enhance further the therapeutic potential of the compositions of the invention, the dual acting polymers can also be formulated to further bind or to keep trapped the ingredient or medication for slow release or to enhance the efficacy.

In another embodiment, the at least one ingredient and/or medication is not bound to the dual acting polymer but remains entrapped between the polymeric linkages of the composition for a sustained release, to further enhance therapeutic properties of the composition. Preferably, said at least one ingredient and/or medication is at least one essential oil.

Also described herein is a method for obtaining such a composition, said method comprising 1) the step of mixing glycerol with the ingredient and/or the medication, and then 2) the step of adding at least one dual acting polymer.

In a preferred embodiment, the method comprises 1) mixing glycerol with at least one essential oil and then 2) adding the at least one dual acting polymer.

The compositions for topical use of the invention are liquid or semi-liquid compositions. These liquid or semi-liquid products can be applied topically as a liquid, inhaler, bandage, solution, gel, cream, paste, or ointment, presented in sprays, tubes, ampoules, liquid embedded cotton or polymeric bandages, or soft-gel capsules to provide the coverage of the targeted surface with a film of the product. The film must remain in contact on the surface of the injury to exert osmotic effects and to continue protecting, hydrating, and cleaning the surface as well as neutralizing selected CPRMs through dual acting polymers.

Oral formulations can be administered as liquid containing soft-gel capsules or as a semi-solid powder or granules containing glycerol and polymeric association in an acceptable base. Then, in a particular embodiment, the compositions of the invention are formulated in liquid or semi-liquid dosage forms containing powder or granules, for single or repeated administration to the subject. In another embodiment, the compositions of the invention are administered as a liquid, inhaler, bandage, solution, gel, cream, paste, or ointment, presented in sprays, tubes, ampoules, liquid embedded cotton or polymeric bandages, granules, powder or soft-gel capsules. To keep the injured surface oxygenated, the bandage protecting the injury should not be airtight.

More commonly these pharmaceutical formulations are prescribed to the patient in "patient packs" containing a number dosing units or other means for administration of metered unit doses, such as sprays, aerosol bottles, sachets, or capsules, for use during a distinct treatment period in a single package, usually as a spray or a tube containing a viscous solution. The invention further includes a pharmaceutical formulation, as herein before described, in combination with packaging material suitable for said formulations.

The composition of the invention is topically applied on a damaged and/or inflammatory biological surface such as the skin, mucosa of the ocular, oral, nasal, gastrointestinal, respiratory, or genital surfaces, for use in the prevention or the treatment of topical inflammatory disease and of the cytokine release syndrome.

Thus, the invention relates to a composition as defined in the appended claims for use in the prevention or the treatment of topical inflammatory disease and for use in preventing or treating CRS. This comprises the topical administration of a composition comprising at least one dual acting polymer bound to glycerol and able to bind to at least one pro-inflammatory compound, to a subject in need thereof.

A therapy according to the invention may be performed in conjunction with any other therapy.

It may be provided at home, the doctor's office, a clinic, a hospital's outpatient department, or a hospital, so that the doctor can observe the therapy's effects closely and make any adjustments that are needed.

The duration of the therapy depends on the stage of the disease being treated, the age and condition of the patient.

The dosage, frequency and mode of administration depends on the stage and the localisation of the disease being treated, the age and condition of the patient, and the sensitivity of the surface where the lesion is located.

### EXAMPLES

### Example 1: selection of dual acting natural polymer

Only those polymers able to bind with glycerol were selected to evaluate their binding properties with pro-inflammatory compounds and disease specific cytokines and/or other proteins and/or cell surface receptor and/or macromolecules (CPRMs). Further, they were evaluated for their cytotoxic potential to check whether the CPRM binding concentration is non-cytotoxic and can be used in acceptable pharmaceutical forms. According to the disease specific topical concentrations of cytokines, other disease related specific proteins, macromolecules or metal ions to be neutralized, the natural polymers were tested against each target molecule (ELISA tests for each proteins, cell culture virus neutralization tests for virus capsid protein neutralization, in *vitro* polymer-metal ion incubation followed by *in vitro* cytotoxicity or *in vivo* blood concentrations for metal ions, and cellular matrix exposure to each MMP followed by cell growth *in vitro* for wound healing tests). The minimum concentrations of each glycerol binding polymer or the best association of the polymers, capable of equally binding and neutralizing maximum disease related CPRMs were then associated with glycerol to render glycerol filmogen for a long-lasting stability when applied topically.

### A - Preparation of natural polymers (tannins)

Eighty-one tannin rich parts of the plant in the form of whole plant (WP), leaves (L), flowers (FI), fruits (F), seeds (S), bark (B), or root (R) were used to prepare tannin rich extracts by maceration (M), Soxhlet (S), or by percolation (P) techniques to collect tannin rich fractions, which were either used as liquid extract or further dried and used as powders. The extraction processes are known to the men of art. In short, to extract the tannin rich fractions by Soxhlet, dried samples (2.5 g) were packed in a cellulose thimble (33×80 mm) (Whatman, GE Healthcare, UK) with Soxhlet apparatus (MS-EAM M-TOP, Indonesia), and extracted with 250 mL of the ethanol for 10 h (one cycle per hour) at 80 °C. Maceration was performed by filling 0.2 g of dried leaf powder in a tightly closed 50 mL glass tube containing 20 mL ethanol, then shaken at 150 rpm for 24 h at room temperature (30±5 °C). Similarly, a percolation column was formed in a disposable syringe (0.5 cm in diameter and 10 cm height) (NIPRO, Japan). A set was 0.1 g of leaf powder packed into the syringe to obtain a 0.2 mL bed volume. The effluent of 10 mL loading was collected as a fraction with the flow rate controlled at 0.1-0.2 mL/min by a vacuum manifold (12-Port Teknokrama, Spain). All the liquid extracts were filtrated through Whatman No. 4 paper, and the volume was made up to compensate for evaporation. Extracts were then evaporated and dried at 60 °C to obtain a dried powder. All samples were kept in separated amber glass bottles with tight stoppers at 4 °C until analysis.

Soluble or partially soluble whole extracts were analysed to quantify plant specific active ingredient for plant identification. These extracts were then used for the preparation of compositions as well as for further pharmacological, analytical, safety, and clinical evaluation of finished compositions.

### B - Selection of synthetic or semi-synthetic polymers

Synthetic polymers can be synthetized from natural tannin base as described by Maria-Fraga *et al*. (2020) or can be synthetized chemically according to the desired binding profile. Synthetic tannins can be purchased from commercial sources such as Sigma Aldrich. Eight key dual acting polymeric structures having glycerol and binding affinity for one of the CPR or M are shown below (Table 2). They were used in different compositions according to their binding specificities in non-cytotoxic concentrations between 0.01% to 5.0% (w/w). The 8 synthetic polymers are listed in Table 2 below.

**Table 2**

| Name | Reference | Provider |
|---|---|---|
| CMCNa - Carboxymethylcellulose under sodium salt (also called Carmellose) | CAS: 9004-32-4 | Sigma-aldrich product code: C5678 |
| PVOH - Poly(vinyl alcohol) 26-88 (PVOH, PVA, or PVAI) Formula: [CH2CH(OH)]n | CAS : 9002-89-5 | Sigma-aldrich product number 1.41352 |
| PCM - Polyacrylamide | CAS 9003-05-8 | Sigma-aldrich product number 92560 |
| SPA- Sodium polyacrylate | CAS 9003-04-7 | Sigma-aldrich product number 432784 |
| PEG - Polyethylene glycol) | CAS 25322-68-3 | Sigma-aldrich product number 81260 |
| PL127 - Pluronic F-127 | CAS: 9003-11-6 | Sigma-aldrich product number P2443 |
| Klucel- Hydroxypropyl cellulose - HPC | Eur. Ph. 9004-64-2 | Ashland ingredient, Wilmington, DE |
| Solagum (association of Acacia gum & Xanthan gum) | | SEPPIC S.A. France |

Only those synthetic polymers which are dual-acting, not absorbed in the body, and act exclusively topically, were used in compositions, either alone or in combination with other natural or semi-synthetic polymers.

### C - Cytotoxicity testing

The maximum non-cytotoxic concentration of each synthetic or natural polymer was evaluated in vitro on 3 different cell cultures (MDBK cell line, MDCK cell line, Chang Liver cell line), purchased from ATCC cell culture collection. In short, cells were grown in 96-well culture plates (90 pL MEM + 10% foetal calf serum) to obtain 100% cell monolayer. Culture medium was discarded and replaced by serum-free MEM containing 0.1%, 0.3%, 1.0%, 2.0% and 5.0% (w/w) concentrations of each test polymer. Cell mortality was evaluated after 72h exposure by counting number of live cells. The maximum test product concentration showing no cellular effects was considered non-cytotoxic. Only those samples showing no cytotoxicity at 2.0% concentration or above were retained for the evaluation of glycerol and specific CPRM binding activities.

### D - Selection of polymer/tannin concentration in the finished composition

We observed that most of the powdered polymers/tannins can be incorporated in the final composition to a concentration of less than 5.0% w/w. Higher concentrations render the composition semi-solid, solid, or opaque which cannot be applied topically as an osmotic film. Therefore, all test products were tested at concentrations of 0.01, 0.03, 0.10%, 0.20%, 0.30%, 1.0%, 2.0% and 3.0%.

The anti-protein activity was evaluated for the following inflammatory and topical disease specific CPRMs: Histamine, IgE, TRP, TSLP, TNF-alpha, IFN-gamma, TGF-beta, IL-1beta, IL-2, IL-4, IL-5, IL-6, IL-8, IL-10, IL-12, IL-13, IL-23, IL-25 IL-33, MMP-1, MMP-2, MMP-3, MMP-8, MMP-9 & MMP-13.

Any dual-acting, natural or synthetic polymer having capacity to bind with one or more disease specific pro-inflammatory compound (CPRM) can be used in compositions. About 15% of the tested polymers showed binding with one or more CPRMs. For example, among commonly used food-grade plant extracts, only the following 14 out of 81 natural polymers showed selective anti-CPRM activities: Vaccinium macrocarpon (Vma) fruit extract (F) extracted through the technique of maceration (M), abbreviated as Vma-F-M; *Camellia sinensis* leaves (Cs-L-M); *Vitis vinifera* seeds (Vv-S-P); *Sambucus nigra* fruits (Sn-F-M); *Hedera helix* leaves (Hh-L-M); *Ribes nigrum* fruits (Rn-F-M); *Curcuma longa* rhizomes (Cl-R-P); *Panax ginseng* roots (Pg-R-M); *Glycine max* seeds (Gm-S-M); *Urtica dioica* whole plant (Ud-WP-M); *Artemisia annua,* (Aa), *Calendula officinalis* (Cao-FI-M); *Salix alba* whole plant (Sa-WP-M); and *Tanacetum parthenium* flowers (Tp-FI-M). They were used purified, not purified as liquid or powdered extract, in pharmaceutically acceptable and non-cytotoxic concentrations.

### Example 2: Selection of glycerol binding tannins

Glycerol cannot be detected by HPLC. Therefore, it was identified with GC-MS (Gas Chromatography Mass Spectrometry). Glycerol can be detected as a single peak between 31-32 min with a pA of 340 (fig. 1-B).

To check the glycerol-polymer binding, HPLC and/or GC-MS profiles of individual polymer were compared with glycerol + polymer profile (fig. 1). The two resulting chromatographic profiles do not show polymer and/or glycerol peaks but a totally different chromatogram showing that all of the polymers incorporated in the composition bind totally with glycerol.

The glycerol binding polymers/tannins were then evaluated to identify their specific CPRPMs binding properties.

### A - Evaluation of cytokine and other protein binding properties of polymers/tannins

Anti-protein activity of 81 non-cytotoxic polymers/tannins rich extracts was evaluated using ELISA (enzyme-linked immunosorbent assay) immunoassay for each protein. Briefly, the ELISA assay is a plate-based assay technique designed for detecting and quantifying peptides, proteins, antibodies, growth factors, and hormones. The sandwich ELISA quantifies antigens between two layers of antibodies (i.e. capture and detection antibody). The antigen to be measured must contain at least two antigenic epitopes capable of binding to antibody, since at least two antibodies act in the sandwich. The detection antibody is linked to an enzyme, and the detection is accomplished by assessing the conjugated enzyme activity via incubation with a substrate to produce a measurable product.

Results are shown in Table 3 below. This Table shows the disease related protein, macromolecule, and / or metal ion binding properties (% inhibition versus controls) of the polymers bound to glycerol in pharmacologically acceptable concentrations. Conc. = Polymer concentration tested, S = Synthetic tannin, EGF= Epithelial growth Factor, FGF = Fibroblast growth factor, MMP= Matrix metalloproteases, - = not performed.

Mean disease related protein binding properties of glycerol binding polymers. Results represent mean of 3 tests at 2 most active and non-cytotoxic concentrations.

Mean disease related protein binding properties of glycerol binding tannins using ELISA tests. Glycerol binding concentration of each test product was incorporated in 30% glycerol solution for testing. Results indicate the absence or presence of dual binding properties of each test product. Results represent mean of 3 tests at 2 most active and non-cytotoxic concentrations of 81 natural or synthetic polymers. Only the results of dual acting polymers (glycerol + one of the disease-related pro-inflammatory compound (CPRM) are presented below.

**Table 3**

| Type of natural or synthetic polymer | Test conc. | Type of CPRM and % inhibition with a specific polymer (natural & synthetic) | | | | | | |
|---|---|---|---|---|---|---|---|---|
| | | Histamine | IgE | TSLP | TNF-α | IFNγ | TGF-β | IL-1β |
| *C. longa* | 0.1 | 49.88 | 93.33 | 31.21 | 9.87 | 43.54 | 84.75 | 17.46 |
| | 0.3 | 80.60 | 95.29 | 20.23 | 15.6 | 58.43 | 98.33 | 39.26 |
| *V. vinifera* | 0.1 | 0.0 | 1.76 | 83.32 | 35.4 | 0.0 | 81.36 | 65.9 |
| | 0.5 | 0.0 | 19.41 | 88.65 | 74.74 | 0.0 | 90.13 | 90.80 |
| *V. myrtillus* | 0.1 | 13.55 | 5.29 | 9.31 | 40.23 | 43.24 | 78.31 | 20.97 |
| | 0.2 | 16.31 | 20.58 | 13.25 | 44.81 | 55.61 | 79.56 | 48.30 |
| *H. helix* | 0.1 | 20.75 | 21.96 | 6.77 | 12.45 | 52.65 | 2.31 | 8.71 |
| | 0.2 | 27.55 | 32.54 | 11.77 | 41.08 | 57.73 | 6.85 | 31.45 |
| *T. parthenium* | 0.1 | 44.24 | 64.31 | 95.69 | 0.0 | 0.0 | 56.35 | 0.35 |
| | 0.2 | 48.85 | 94.5 | 99.80 | 0.0 | 0.0 | 68.11 | 40.50 |
| *U. dioica* | 0.1 | 9.87 | 8.62 | 19.27 | 12.3 | 38.58 | 14.66 | 9.17 |
| | 0.3 | 19.14 | 8.43 | 45.66 | 36.9 | 40.55 | 12.68 | 32.68 |
| *Artemisia annua* | 0.03 | 0 | 0 | 0 | 32.65 | 56.34 | 0 | 0 |
| | 0.1 | 0 | 0 | 0 | 44.91 | 62.39 | 0 | 0 |
| *C.sinensis* | 0.1 | 51.3 | 6.4 | 0.0 | 6.3 | 71.3 | 3.11 | 51.88 |
| | 0.3 | 80.5 | 7.2 | 0.0 | 7.7 | 87.4 | 5.64 | 62.96 |
| *C. officinalis* | 0.1 | 21.3 | 16.1 | 0.0 | 1.59 | 42.06 | 0.0 | 0.40 |
| | 0.3 | 17.5 | 14.1 | 0.0 | 0.0 | 47.98 | 2.06 | 11.87 |
| *P. ginseng* | 0.1 | 0.7 | 0.7 | 0.0 | 52.9 | 0.0 | 3.2 | 1.2 |
| | 0.5 | 37.4 | 25.8 | 0.0 | 61.6 | 0.0 | 2.98 | 0.0 |
| *V*. *macrocarpon* | 0.1 | 5.5 | 8.3 | 15.36 | 0.30 | 37.84 | 96.97 | 42.42 |
| | 0.5 | 12.1 | 10.2 | 22.15 | 45.41 | 57075 | 99.56 | 77.93 |
| *S. nigra* | 0.1 | 3.2 | 3.9 | 0.0 | 4.3 | 0.0 | 66.3 | 77.32 |
| | 0.5 | 11.0 | 4.5 | 0.0 | 14.37 | 0.0 | 59.34 | 51.02 |
| *S. alba* | 0.3 | 5.4 | 2.5 | 0.0 | 16.20 | 0.0 | 81.3 | 23.64 |
| | 0.5 | 0.0 | 0.5 | 0.0 | 66.74 | 0.0 | 90.20 | 14.36 |
| Solagum | 0.5 | 8.7 | 52.5 | 26.66 | 0.0 | 0.0 | 0.0 | 0.0 |
| *R. Nigrum* | 0.3 | 0.0 | 0.0 | 0.0 | 0.0 | 47.39 | 0.0 | 0.0 |
| | 0.5 | 0.0 | 0.0 | 0.0 | 0.0 | 70.26 | 0.0 | 0.0 |
| CMCNa (S) | 0.1 | 0.0 | 49.3 | 0.0 | 0.0 | 14.84 | 0.0 | 2.16 |
| PVOH | 0.2 | 1.2 | 6.1 | - | 44.1 | - | 81.6 | 3.44 |
| SPA | 0.2 | 36.7 | 32.3 | - | 2.1 | - | 3.47 | 5.98 |
| PL127 | 0.1 | 22.1 | 4.0 | 0.0 | 67.4 | - | 0.0 | 56.3 |
| HPC | 0.5 | 26.4 | 2.4 | 0.0 | 74.12 | 4.36 | 0.5 | 29.34 |

**Table 3 (continued)**

| Type of natural or synthetic polymer | Test conc. | Type of CPRM and % inhibition with a specific polymer (natural & synthetic) | | | | | |
|---|---|---|---|---|---|---|---|
| | | MMP-1 | MMP-2 | MMP-3 | MMP-8 | MMP-9 | MMP-13 |
| *C. longa* | 0.1 | 0.0 | 0.0 | 0.0 | 3.11 | 0.0 | 2.45 |
| | 0.3 | 0.0 | 0.0 | 0.0 | 15.15 | 0.0 | 6.04 |
| *V. vinifera* | 0.1 | 34.61 | 54.64 | 5.69 | 6.15 | 34.79 | 38.88 |
| | 0.5 | 65.27 | 14.12 | 46.31 | 11.79 | 28.86 | 42.0 |
| *V. myrtillus* | 0.1 | 22.73 | 10.02 | 74.41 | 6.99 | 54.08 | 52.94 |
| | 0.2 | 46.72 | 20.99 | 79.81 | 12.81 | 75.07 | 65.50 |
| *H. helix* | 0.1 | 0.0 | 0.0 | 63.70 | 3.21 | 0.0 | 0.0 |
| | 0.2 | 0.0 | 0.0 | 83.14 | 9.31 | 0.0 | 0.0 |
| *T. parthenium* | 0.1 | 6.35 | 0.0 | 0.0 | 6.31 | 0.0 | 6.32 |
| | 0.2 | 14.36 | 0.0 | 0.0 | 15.02 | 0.0 | 16.0 |
| *U. dioica* | 0.1 | 0.0 | 3.69 | 0.0 | 5.34 | 0.0 | 0.0 |
| | 0.3 | 0.0 | 12.41 | 0.0 | 5.11 | 0.0 | 2.11 |
| *Artemisia annua* | 0.03 | 0 | 0 | 7.35 | 2.98 | 0 | 0 |
| | 0.1 | 0 | 0 | 11.33 | 15.51 | 0 | 0 |
| *C.sinensis* | 0.1 | 98.01 | 39.52 | 2.31 | 54.44 | 57.89 | 35.31 |
| | 0.3 | 99.63 | 71.24 | 3.63 | 73.98 | 91.39 | 68.10 |
| *C. officinalis* | 0.1 | 0.0 | 0.0 | 94.71 | 2.41 | 0.0 | 0.0 |
| | 0.3 | 0.0 | 0.0 | 97.85 | 14.96 | 0.0 | 0.0 |
| *P. ginseng* | 0.1 | 0.0 | 0.0 | - | - | 0.0 | 0.0 |
| | 0.5 | 0.0 | 0.0 | - | - | 0.0 | 0.0 |
| *V. macrocarpon* | 0.1 | 45.68 | 50.85 | 0.0 | 56.25 | 7.11 | 0.10 |
| | 0.5 | 59.40 | 60.52 | 0.5 | 61.39 | 35.95 | 12.27 |
| *S. nigra* | 0.1 | 0.0 | 0.0 | 0.0 | 6.54 | 0.0 | 0.0 |
| | 0.5 | 0.0 | - | 0.0 | 7.21 | 0.0 | 4.19 |
| *S. alba* | 0.3 | 0.0 | - | 0.0 | 32.33 | 3.69 | 0.0 |
| | 0.5 | 0.0 | - | 0.0 | 42.94 | 4.56 | 0.0 |
| Solagum | 0.5 | 0.0 | 0.0 | 0.0 | 26.39 | 0.0 | 36.56 |
| *R. Nigrum* | 0.3 | 9.22 | 13.04 | 21.96 | 0.0 | 0.00.0 | 0.0 |
| | 0.5 | 99.61 | 72.67 | 98.19 | 0.0 | 9.88 | 0.0 |
| CMCNa (S) | 0.1 | 0.0 | 13.69 | 0.0 | 6.44 | - | 0.0 |
| PVOH | 0.2 | 0.0 | - | 2.396 | 36.4 | - | 0.0 |
| SPA | 0.2 | 0.0 | - | 0.0 | 0.0 | 54.12 | 0.0 |
| PL127 | 0.1 | 0.0 | - | 0.0 | 61.6 | 0.0 | 26.21 |
| HPC | 0.5 | 0.0 | - | 0.0 | 65.55 | 0.0 | 0.0 |

**Table 3 (continued)**

| Type of natural or synthetic polymer | Test conc. | Type of CPRM and % inhibition with a specific polymer (natural & synthetic) | | | | | | | | | |
|---|---|---|---|---|---|---|---|---|---|---|---|
| | | IL-2 | IL-4 | IL-5 | IL-6 | IL-10 | IL-12 | IL-13 | IL-23 | IL-25 | IL-33 |
| *C. longa* | 0.1 | 47.41 | 4.20 | 46.27 | 0.2 | 3.79 | 36.55 | 3.49 | 35.94 | 22.14 | 46.36 |
| | 0.3 | 90.11 | 5.49 | 42.34 | 15.76 | 0.10 | 47.90 | 7.74 | 66.33 | 12.81 | 49.80 |
| *V. vinifera* | 0.1 | 8.47 | 52.12 | 8.72 | 31.2 | 0.0 | 69.57 | 13.63 | 58.03 | 99.43 | 4.20 |
| | 0.5 | 13.21 | 73.93 | 0.53 | 49.44 | 0.0 | 83.28 | 28.15 | 63.54 | 85.56 | 7.98 |
| *V. myrtillus* | 0.1 | 23.64 | 10.0 | 0.0 | 7.92 | 4.04 | 71.65 | 1.39 | 30.89 | 0.85 | 0.66 |
| | 0.2 | 40.43 | 15.67 | 15.06 | 31.68 | 6.62 | 64.92 | 23.88 | 62.58 | 8.63 | 0.26 |
| *H. helix* | 0.1 | 0.0 | 0.88 | 26.04 | 0.3 | 2.04 | 12.36 | 42.72 | 0.0 | 1.66 | 5.45 |
| | 0.2 | 0.0 | 2.04 | 2.03 | 19.83 | 0.0 | 14.38 | 96.75 | 0.0 | 2.23 | 10.36 |
| *T. parthenium* | 0.1 | 0.0 | 34.36 | 1.71 | 0.4 | 0.0 | 36.31 | 34.52 | 16.35 | 62.53 | 17.68 |
| | 0.2 | 0.0 | 53.03 | 9.81 | 32.16 | 0.0 | 47.44 | 99.60 | 24.98 | 86.83 | 41.15 |
| *U. dioica* | 0.1 | 1.12 | 19.63 | 13.33 | 0.3 | 5.38 | 0.0 | 20.4 | 0.0 | 25.69 | 44.32 |
| | 0.3 | 0.0 | 3.57 | 14.60 | 10.27 | 4.61 | 0.0 | 80.85 | 0.0 | 34.19 | 54.51 |
| *A. annua* | 0.03 | 6.85 | 0 | 11.36 | 68.39 | 55.88 | 0 | 0 | 13.49 | 3.56 | 8.87 |
| | 0.1 | 5.96 | 0 | 25.14 | 74.44 | 53.69 | 0 | 0 | 18.32 | 14.98 | 11.36 |
| *C. sinensis* | 0.1 | 56.9 | 76.6 | 36.82 | 44.69 | 6.28 | 0.0 | 78.2 | 42.07 | - | 0.0 |
| | 0.3 | 90.11 | 88.4 | 92.4 | 60.97 | 6.46 | 0.0 | 81.8 | 51.65 | - | 0.0 |
| *C. officinalis* | 0.1 | 48.36 | 31.5 | 28.4 | 0.30 | 2.28 | 12.9 | 1.5 | 9.68 | - | 0.0 |
| | 0.3 | 42.89 | 51.9 | 49.2 | 0.20 | 0.0 | 36.44 | 3.8 | 15.31 | - | 0.0 |
| *P. ginseng* | 0.1 | 0.0 | 0.0 | 3.6 | 0.0 | | 2.56 | 42.1 | 0.0 | 4.32 | 0.0 |
| | 0.5 | 0.0 | 0.0 | 11.0 | 6.1 | | 2.74 | 36.6 | 0.0 | 16.69 | 0.0 |
| *V*. *macrocarpon* | 0.1 | 36.41 | 32.2 | 28.2 | 3.07 | 0.1 | 16.35 | 0.1 | 20.27 | - | 3.69 |
| | 0.5 | 40.43 | 66.3 | 55.2 | 26.68 | 14.22 | 19.17 | 17.2 | 17.36 | - | 21.25 |
| *S. nigra* | 0.1 | 36.30 | 0.0 | 36.2 | 0.0 | 0.0 | 31.54 | 22.3 | 36.87 | - | 0.0 |
| | 0.5 | 51.83 | 0.0 | 31.7 | 0.0 | 0.0 | 14.32 | 68.1 | 44.58 | - | 0.0 |
| *S. alba* | 0.3 | 0.0 | 2.6 | 26.3 | 25.3 | 0.0 | 71.56 | 81.6 | 47.80 | - | 0.0 |
| | 0.5 | 0.0 | 2.1 | 22.1 | 39.68 | 0.0 | 84.50 | 84.6 | 59.36 | - | 0.0 |
| *R. nigrum* | 0.3 | - | 0.0 | 0.0 | 0.0 | 1.21 | 0.0 | 0.0 | 0.0 | - | 0.0 |
| | 0.5 | - | 0.0 | 0.0 | 0.0 | 14.0 | 0.0 | 0.0 | 9.45 | - | 0.0 |
| CMCNa (S) | 0.5 | 0.0 | 4.5 | 36.6 | 0.0 | 0.0 | 33.21 | 6.6 | 12.69 | - | 3.69 |
| PVOH | 0.1 | 0.0 | 0.0 | 0.0 | 0.0 | 0.0 | 25.58 | 54.2 | 0.0 | - | 12.12 |
| SPA | 0.2 | 0.0 | 0.0 | 44.5 | 0.0 | 4.58 | 0.0 | 2.4 | 0.0 | - | 0.0 |
| PL127 | 0.2 | 0.0 | 12.3 | 2.1 | 0.0 | 0.0 | 16.37 | 54.1 | 3.48 | - | 0.0 |
| HPC | 0.1 | 0.0 | 0.0 | 3.8 | 41.6 | 0.0 | 18.69 | 0.0 | 0.0 | - | 2.22 |

**Table 3 (continued)**

| | | | | | | |
|---|---|---|---|---|---|---|
| Type of natural or | Test conc. | Type of CPRM and % inhibition with a specific polymer (natural & synthetic) | | | | |

| synthetic polymer | | Infl. virus | Herpes virus | As | Hg | TRP receptor protein |
|---|---|---|---|---|---|---|
| *C. longa* | 0.1 | 11.58 | 7.62 | 23.41 | 14.33 | 66.72 |
| | 0.3 | 39.26 | 26.42 | 34.34 | 21.34 | 98.44 |
| *V. vinifera* | 0.1 | 6.63 | 2.49 | 0.0 | 0.0 | 0.0 |
| | 0.5 | 16.91 | 32.50 | 0.0 | 0.0 | 0.0 |
| *V. myrtillus* | 0.1 | 5.12 | 3.14 | 3.21 | 27.30 | 0.0 |
| | 0.2 | 24.72 | 17.16 | 2.11 | 24.69 | 0.0 |
| *H. helix* | 0.1 | 8.66 | 9.09 | 36.58 | 23.56 | 68.59 |
| | 0.2 | 16.85 | 40.72 | 41.78 | 25.11 | 82.41 |
| *T. parthenium* | 0.1 | 2.01 | 0.0 | 0.0 | 0.0 | 61.39 |
| | 0.2 | 0.0 | 0.0 | 0.0 | 0.0 | 78.21 |
| *U. dioica* | 0.1 | 3.59 | 8.20 | 16.22 | 17.29 | 6.56 |
| | 0.3 | 6.42 | 4.21 | 24.31 | 27.89 | 7.42 |
| *Artemisia annua* | 0.03 | 6.32 | 25.67 | 0 | 21.24 | 0.0 |
| | 0.1 | 5.47 | 33.23 | 0 | 76.36 | 0.0 |
| *C. sinensis* | 0.1 | 11.86 | 7.72 | 12.14 | 9.08 | 55.21 |
| | 0.3 | 30.19 | 32.22 | 39.20 | 21.18 | 28.42 |
| *C. officinalis* | 0.1 | 0.0 | 0.0 | 0.0 | 0.0 | 3.53 |
| | 0.3 | 0.0 | 0.0 | 0.0 | 0.0 | 38.00 |
| *P. ginseng* | 0.1 | 1.22 | 1.59 | 0.0 | 0.0 | 5.05 |
| | 0.5 | 2.11 | 6.31 | 0.0 | 0.0 | 6.59 |
| *V*. *macrocarpon* | 0.1 | 18.96 | 12.76 | 0.0 | 0.0 | 0.0 |
| | 0.5 | 26.84 | 29.26 | 0.0 | 0.0 | 0.0 |
| *S. nigra* | 0.1 | 13.26 | 8.71 | 0.0 | 3.46 | 68.74 |
| | 0.5 | 42.55 | 18.64 | 0.0 | 16.24 | 5.93 |
| *S. alba* | 0.3 | 0.0 | 0.0 | 7.22 | 0.0 | 11.1 |
| | 0.5 | 0.0 | 0.0 | 31.26 | 0.0 | 8.50 |
| *R. nigrum* | 0.3 | 14.52 | 7.62 | 0.0 | 9.87 | 0.0 |
| | 0.5 | 24.55 | 16.61 | 0.0 | 28.46 | 6.23 |
| CMCNa (S) | 0.5 | 0.0 | 0.0 | 0.0 | 0.0 | 46.32 |
| PVOH | 0.1 | 0.0 | 0.0 | 0.0 | 0.0 | 61.02 |
| SPA | 0.2 | 0.0 | 0.0 | 11.36 | 7.36 | 0.0 |
| PL127 | 0.2 | 33.84 | 16.56 | 0.0 | 0.0 | 0.0 |
| HPC | 0.1 | 0.0 | 24.36 | 0.0 | 0.0 | 0.0 |

| | | | | | | |
|---|---|---|---|---|---|---|
| Note: Due to polymeric agglutination, occasionally higher concentrations (cone.) may be less effective. All other polymers have shown an inhibition of less than 20% on key disease related proteins or macromolecules or metal ions. | | | | | | |

Conclusion: The protein, macromolecule or metal ion binding properties of 81 selected natural or synthetic glycerol binding polymers were evaluated by ELISA at different concentrations. The results are presented for concentrations below 0.5% which are used to prepare compositions. Among 81 test products, 15 natural and 5 synthetic samples showed an inhibition of more than 20% of one or more proteins, macromolecules or metal ions. The results show that (1) The dual acting polymer - protein, macromolecule, or metal ion binding is highly specific as all polymers do not bind with all the molecules. (2) One dual acting polymer may bind with more than one protein. (3) According to the disease specific protein, macromolecule or metal ion to be neutralized, the dual acting polymers were associated in pharmacologically acceptable concentrations to maximize antagonist effects.

### B - Evaluation of virus glycoprotein binding properties of tannins

The antiviral activity of key natural or synthetic polymers was determined *in vitro* cell cultures by quantifying virus induced cell death. Vero cells for herpes virus and MDCK host cell cultures for influenza virus were grown in 96-well tissue culture plates (37°C - 5% CO2). Minimum virus concentrations capable of inducing 100% cell death (Tissue Culture Infective Dose: TCID100) in 72h was determined using MTT vital cell stain measuring optical density at 560 nm. Twenty-seven non-cytotoxic, glycerol binding polymers at concentration between 0.10% and 0.30% were individually incubated with 0.9% NaCl solution or with 30% filmogen glycerol for 1h. and exposed to the host cell cultures for 72h and virus induced cell death was measured. Reduction in cell death was due to polymer-virus capsid protein binding and in consequence virus neutralization. The polymers showing anti-viral activity were then associated with each other (0.10% each) and the experiments were repeated (n=3; 16 well per experiment). Results are expressed as % increase (+) or decrease (-) in virus growth compared to (TCID100) untreated virus controls. Results are shown in Table 4 below (Vma = *Vaccinium macrocarpon,* Vmy: *Vaccinium myrtillus,* Vv = *Vitis vinifera* seeds, Sn = *Sambucus nigra,* Hh = *Hedera helix,* Rn = *Ribes nigrum,* Cl = *Curcuma longa,* Co = *Calendula officinalis,* Ud = *Urtica dioica,* Cs = *Camellia sinensis,* CMCNa = Carboxymethylcellulose sodium salt, PL127 = Ploronic F-127, HPC = Hydroxypropyl cellulose). Decreased growth (-) is proportional to virus inhibition %.

**Table 4: decrease in virus growth (%) for glycerol binding polymer (0.10%) in saline (control) or in glycerol.**

| S. No. | Polymers and combinations | Polymers in saline | | Polymers in 30% glycerol | |
|---|---|---|---|---|---|
| | | Herpes | Influenza | Herpes | Influenza |
| 1 | Cl | -7,62±1,08 | -11,58±2,29 | -26,42±4,27* | -39,26±2,97 |
| 2 | Vv | -2,49±3,96 | -6,63±3,13 | -32,50±3,40* | -16,91±2,49* |
| 3 | Vma | -12,76±3,63 | -18,96±2,76 | -29,26±3,72* | -26,84±4,1* |
| 4 | Hh | -16,49±3,19 | 11,84±2,85 | -30,81±3,36 | -27,56±4,56* |
| 5 | Vmy | +3,14±4,72 | +5,12±3,75 | -17,16±7,23* | -24,72±3,32* |
| 6 | Rn | -7,62±3,8 | -14,52±2,52 | -16,61±3,70* | -24,55±2,95 |
| 7 | Cs | -7,72±3,83 | -11,86±3,08 | -32,22±3,97* | -30,19±3,07* |
| 8 | Sn | +8,71±3,75 | -13,26±2.03 | -18,64±4,87* | -42,55±3,64* |
| 9 | CMCNa (s) | -2,82±3,1 | -10,48±2,52 | -9.3-±13,70* | -27,56±2,95 |
| 10 | PL127 (s) | +1,78±2,63 | -12,16±3,80 | -32,22±3,97* | -23,10±2,17 |
| 11 | HPC (s) | 9,11±1,85 | -10,03±2.21 | -13,44±3,12 | -21,35±2,33 |
| 12 | Ud | -8,20±2,8 | +3,59±1.69 | -04,21±1,72 | -6,42±2,35 |
| 13 | Sn + Vma | -19,12±3,43 | -19,96±2,88 | -86,31±3,43* | -48,36±3,43 |
| 14 | Sn +Vmy | -31,24±2,39 | -24,33±3,38 | -91,44±3,29* | -50,48±2,6 |
| 15 | Sn + Vv | -24,56±3,2 | -18,39±2,92 | -86,39±4,40* | -61,37±2,19* |
| 16 | Sn + Hh | -18,88±2,55 | -17,22±4,01 | -81,77±4,91* | -33,49±3,87 |
| 17 | Vma + Vmy | -12,48±3,89 | -8,61±2,62 | -38,41±4,70 | -67,11±2,44* |
| 18 | Cs + Vv | -20,01±3,65 | -24,46±2,21 | -41,72±5,88* | -89,36±2,51* |
| 19 | Cs + Sn | +7,26±3,41 | -19,38±2,56 | -64,16±3,75* | -52,30±2,93* |
| 20 | Cl +Co | -11,91±4,47 | -26,64±1,95 | -78,37±4,01* | -81,13±2,41* |
| 21 | Cs + CMCNa | -22,03±2.31 | -14,21±3.68 | -41,30±391 | -58.91±3.25* |
| 22 | Cs + PL127 | -28.31±4.01 | -9.36±2.11 | -43.58±2,28 | -36.32±3,53 |
| 23 | Cs + HPC | +18,16±2,21 | -26.32±3.74 | -71,26±3.55* | -72,13±3,22* |
| 24 | Cl +Co | -11,91±4,47 | -26,64±1,95 | -78,37±4,01* | -81,13±2,41* |
| 25 | Hh + Vv | +8,64±4,49 | -22,37±1,96 | -81,52±4,63* | -44,76±2,32 |
| 26 | Rn + Vv | -17,54±4,07 | -34,56±1,84 | -43,39±4,25* | -65,91±1,80 |
| 27 | Rn + Vma | -22,46±3,52 | +14,71±2,65 | -78,31±4,22* | -29,55±3,22 |
| 28 | Rn + Vmy | -16,98±2,90 | -27,52±1,82 | -36,31±5,21 | -82,15±1,83* |
| Others | Other 2 or 3 polymers associations | < -10,0% | < -10,0% | < -10,0% | < 10,0% |

After initial experiments, 12 of the 32 polymers and their associations showed moderate to strong antiviral activity against influenza (H1N1) or herpes viruses. The dual acting polymers were Vma, Vmy, Vv, Sn, Hh, Rn, Cl, Co, Ud, Cs, CMCNa, PL127 and HPC.

Results indicate that: (1) only 12 among 32 polymers neutralized above 5% virus, (2) The antiviral activity is specific as some polymers neutralizing herpes virus were not effective against influenza virus, (3) Associations of some specific polymers are more effective vs individual polymer, (4) The antiviral activity is much stronger when antiviral polymers were pre-incubated with filmogen glycerol, and (5) filmogen glycerol enhances antiviral activity probably by presenting the polymer-virus binding sites in a better configuration.

### C - Evaluation of metal ion binding properties

The aim of these studies was to evaluate the binding and neutralization of arsenic (As) and cadmium (Cd) metal ions with or without prior incubation with the natural or synthetic tannins at 2 different concentrations, on 3 different cell lines *in vitro.* The cell lines were MDBK (Madin-Darby Bovine Kidney) epithelial cell line of bovine kidneys, CHANG LIVER: Liver human cell line, and HeLa (Human cervical cancer immortal cell line).

Cadmium (CdCl2) and arsenic (NaAs02) were purchased from Sigma (UK). The metals were mixed in culture medium (MEM) at different concentrations between 10⁻⁴ to 10⁻⁷ stock solution concentrations. Ten µL of this solution was then added in 90 µ! culture medium to obtain final concentration for testing. According to the cellular toxicity results observed, the concentrations were further refined to identify the concentration which induces nearly 50% cell death (± 4.0%). Once the 50% cell death concentration of As and Cd were determined (CT₅₀ concentrations), these concentrations were employed for further experiments (to mix with polymers or as positive controls).

### D - Metal ion - polymer binding

Different concentrations of each polymer in a composition comprising glycerol was incubated with CT₅₀ concentration of the metal ions for 1h at 37°C and exposed to cell lines. If the metals are bound by polymers, they cannot exert cytotoxicity. Reduction in cytotoxicity (n=3, minimum 8 wells per concentration) was determined. The reduction % is proportional to % neutralization of the metal ion.

Seven 7 polymeric compositions (Cl, Vmy, CMCNa, HPC, Hh, Ud, and Cs) were found to neutralize up to 60% of metal ions. Different polymeric associations were prepared to block up to 90% metals. These polymer(s) were used in topical compositions requiring neutralization of heavy metals.

Mercury (Hg) being a volatile metal, Hg-polymer binding was determined in rats. All key polymers and their associations were tested. A group of rats (6+6/ group) was fed with 1 mg/kg Hg, a second group with 4 mg/kg, a third group with 4 mg/Hg and 300 mg/kg of dual acting polymer(s) and a fourth group was fed as the third group except the dual acting polymer(s)-Hg association(s) was preincubated for 1h at room temperature. Animals were sacrificed after 14 days repeated dosing and the amount of Hg vapours in the blood was measured. With one of the polymers associations (Cl + Hh), the animals of third group showed 31.26% less Hg in the blood and the animals in fourth group had 94.30% less Hg in the blood compared to the group 2 Hg controls. These results indicate that Hg can also be neutralized with specific polymer and polymeric associations.

### Conception of dual acting compositions for the treatment of specific topical diseases

All compositions contain glycerol with one or more dual acting polymer/tannin, i.e. a polymer/tannin capable of binding with glycerol (filmogen glycerol) and with one or more disease specific CPRMs.

Throat sprays were filled in 30 mL aluminium containers for adults and 20 mL aluminium containers for children, fitted with a spray for topical application. Posology: 3-4 sprays on the throat surface, 2-3 times per day for a maximum period of 14 days.

Nasal sprays for the treatment of rhinitis, pollution allergy, and sinusitis was filled in 15 mL plastic containers for adults and children, fitted with a spray for topical application. Posology: 3-4 sprays on the nasal surface, 2-3 times per day for a maximum period of 14 days. Allergy treatments were applied whenever required during exposure to the allergen (symptomatic treatment) or 15 min before exposure to the allergen (preventive treatment).

The products for topical application on the skin, wounds, haemorrhoids, herpes, and genital infections were filled in 10 mL or 50 mL plastic tubes. Depending upon the surface of the lesion, quantities sufficient to form a thin product film on the damaged surface were applied, 5-6 times per day, directly on the injured surface.

To treat gastric ulcers, 5 mL of product solution was administered orally, 4 times a day for a period of maximum 14 days.

To treat gastrointestinal inflammation, 10% filmogen glycerol containing 2 polymeric extracts was mixed with ispaghula seed tegument as excipient and the powder was filled in 5g sachets. The same composition was also formulated as granules. 5g product was administered orally, once a day for a period of 1 month and repeated whenever necessary.

To treat internal haemorrhoids, 1 g of viscous product was filled in gelatine suppositories and was administered as 1 suppository in the morning and one in the evening for a period of 6 consecutive weeks.

Topical gel for the treatment of skin infections containing filmogen glycerol with essential oils was also filled in 30 mL glass vials fitted with a roll-on applicator. The product was applied twice a day for a maximum period of 1 month.

All the compositions contained dual acting natural or synthetic specific CPRM binding polymers to render the glycerol filmogen and simultaneously to bind with one or more disease specific CPRMs.

A very small quantity of essential oils was added in some compositions as an excipient to improve the smell.

A few examples of compositions according to the invention, that show a beneficial effect in treating topical inflammatory diseases, are given below:
- Glycerol 74.94%, honey 13.57%, water 10.76%, *Vitis vinifera* extract 0.44%, *Sambucus nigra* extract 0.29%, particularly suitable for the treatment of throat infection.
- Glycerol 74.94%, water 16.76%, honey 7.57%, CMCNa 0.30%, *Sambucus nigra* extract 0.43, particularly suitable for the treatment of throat infection.
- Glycerol 62%, water 31.19%, honey 6%, HPC 0.15%, PL127 0.35%, PEG 0.23%, *Mentha piperita* essential oil 0.02%, *Eucalyptus globulus* essential oil 0.02%, *Rosmarinus officinalis* essential oil 0.02%, *Thymus satureioides* essential oil 0.02%, particularly suitable for the treatment of throat pain.
- Glycerol 62%, water 36.62%, *Hedera helix* extract 0.96%, *Ribes nigrum* extract 0.36%, *Mentha piperita* essential oil 0.02%, *Rosmarinus officinalis* essential oil 0.02%, *Thymus satureioides* essential oil 0.02%, particularly suitable for the treatment of throat pain and inflammation.
- Glycerol 49.20%, water 50.68%, PCM 0.02%, *Curcuma longa* extract 0.08%, *Eucalyptus globulus* essential oil 0.02%, particularly suitable for the treatment of wet cough.
- Glycerol 40%, water 50.43%, honey 8%, *Calendula officinalis* extract 0.5%, *Eucalyptus globulus* essential oil 0.02%, *Linum usitatissimum* oil 0.05%, propolis extract 1%, particularly suitable for the treatment of dry cough.
- Glycerol 37.37%, water 62.04%, *Vaccinium macrocarpon* extract 0.13%, *Camellia sinensis* extract 0.23%, *Vaccinium myrtillus* extract 0.13%, *Sambucus nigra* extract 0.1%, particularly suitable for the treatment of nasal infection (rhinosinusitis).
- Glycerol 26.14%, water 73.2%, HPC 0.5%, *Mentha piperita* essential oil 0.04%, *Eucalyptus globulus* essential oil 0.04%, *Rosmarinus officinalis* essential oil 0.04%, *Thymus satureioides* essential oil 0.04%, particularly suitable for the treatment of nasal infection (rhinosinusitis).
- Glycerol 26%, water 73.02%, HPC 0.5%, *Vaccinium macrocarpon* extract 0.07%, *Camellia sinensis* extract 0.13%, *Vaccinium myrtillus* extract 0.07%, SPA 0.21%, particularly suitable for the treatment of nasal infection (rhinosinusitis).
- Glycerol 9.8%, water 89.317%, Solagum 0.5%, *Ribes nigrum* extract 0.05%, *Curcuma longa* extract 0.08%, *Mentha piperita* essential oil 0.05%, *Eucalyptus globulus* essential oil 0.05%, *Rosmarinus officinalis* essential oil 0.05%, *Thymus satureioides* essential oil 0.05%, preservatives 0.053% (potassium sorbate 0.025%, sodium benzoate 0.025%, citric acid 0.003%), particularly suitable for the treatment of nasal infection (rhino-sinusitis)
- Glycerol 2%, water 96.9%, Solagum 0.5%, *Camellia sinensis* extract 0.09%, *Curcuma longa* extract 0.08%, *Panax ginseng* extract 0.06%, *Urtica dioica* extract 0.1%, *Citrus limonum* essential oil 0.05%, preservatives 0.22% (potassium sorbate 0.1%, sodium benzoate 0.1%, citric acid 0.02%), particularly suitable for the treatment of allergic rhinitis.
- Glycerol 3%, water 95.84%, HPC 0.55%, *Vaccinium myrtillus* extract 0.1%, *Hedera helix* extract 0.13%, *Curcuma longa* extract 0.07%, *Mentha piperita* essential oil 0.08%, preservatives 0.23% (potassium sorbate 0.1%, sodium benzoate 0.1%, citric acid 0.03%), particularly suitable for the treatment of pollution induced allergy.
- Glycerol 9.8%, water 89.15%, PL127 0.25%, HPC 0.25%, *Camellia sinensis* extract 0.08%, *Curcuma longa* extract 0.07%, *Urtica dioica* extract 0.08%, *Tanacetum parthenium* extract 0.1%, preservatives 0.22% (potassium sorbate 0.1%, sodium benzoate 0.1%, citric acid 0.02%), particularly suitable for the treatment of respiratory symptoms induced by pollution and allergens.
- Glycerol 74.63%, water 11%, honey 13.58%, *Vitis vinifera* extract 0.44%, *Sambucus nigra* extract 0.29%, *Glycine max* extract 0.06%, particularly suitable for the treatment of throat infection in children.
- Glycerol 74.63%, honey 6%, water 18.54%, *Vitis vinifera* extract 0.44%, *Sambucus nigra* extract 0.29%, *Ribes nigrum* extract 0.1%, particularly suitable for the treatment of throat infection and pain in children.
- Glycerol 53%, water 41.81%, honey 4.5%, *Ribes nigrum* extract 0.36%, *Curcuma longa* extract 0.08%, *Citrus limonum* essential oil 0.02%, preservatives 0.23% (potassium sorbate 0.1%, sodium benzoate 0.1%, citric acid 0.03%), particularly suitable for the treatment of cough in children.
- Glycerol 13%, water 85.96%, HPC 0.5%, *Vaccinium macrocarpon* extract 0.07%, *Vaccinium myrtillus* extract 0.07%, *Sambucus nigra* extract 0.05%, *Ribes nigrum* extract 0.13%, preservatives 0.22% (potassium sorbate 0.1%, sodium benzoate 0.1%, citric acid 0.02%), particularly suitable for the treatment of nasal infection in children.
- Glycerol 4%, water 94.87%, Solagum 0.6%, *Vaccinium macrocarpon* extract 0.1%, PVOH 0.1%, *Panax ginseng* extract 0.06%, *Thymus satureioides* essential oil 0.03%, *Citrus limonum* essential oil 0.02%, preservatives 0.22% (potassium sorbate 0.1%, sodium benzoate 0.1%, citric acid 0.02%), particularly suitable for the treatment of allergic rhinitis in children.
- Glycerol 67.04%, water 1.15%, honey 31.41%, xanthan gum 0.08%, *Vaccinium macrocarpon* extract 0.16%, *Vaccinium myrtillus* extract 0.16%, particularly suitable for the treatment of oral ulcers.
- Glycerol 67.04%, water 1.15%, honey 31.41%, xanthan gum 0.08%, *Vaccinium macrocarpon* extract 0.16%, *Vaccinium myrtillus* extract 0.16%, particularly suitable for the treatment of gastric ulcers.
- Glycerol 73.85%, water 11.94%, honey 13.6%, *Vaccinium macrocarpon* extract 0.28%, *Vaccinium myrtillus* extract 0.33%, particularly suitable for the treatment of chemotherapy induced oral mucositis.
- Glycerol 66.15%, water25.50%, honey 5.85%, *Vaccinium macrocarpon* extract 1.5%, *Vaccinium myrtillus* extract1.30%, *Artemisia annua* 0.70%, particularly suitable for the treatment of traumatic infected oral mucositis.
- Glycerol 66.52%, water 1.54%, honey 31.44%, xanthan gum 0.08%, *Camellia sinensis* extract 0.26%, *Vaccinium myrtillus* extract 0.16%, particularly suitable for the treatment of chronic wound.
- Glycerol 7.70%, water 91.56%, HPC 0.60%, *Hedera helix* extract 0.04%, *Curcuma longa* extract 0.20%, *Tanacetum parthenium* 0.08%, particularly suitable for the treatment of ocular allergy.
- Glycerol 21.0%, water 78.20%, Solagum 0.50%, *Vaccinium macrocarpon* 0.14%, *Vaccinium myrtillus* extract 0.16%, particularly suitable for the treatment of eye dryness.
- Glycerol 13.6%, water 86.0%, PL127 0.25%, *Tanacetum parthenium* extract 0.14%, Papaya - Folium Eriobotryae gum extract 0.01%, particularly suitable for the treatment of diabetic retinopathy.
- Glycerol 7.70%, water 91.97%, HPC 0.20%, *Urtica dioica* extract 0.05%, *Vitis vinifera* seed extract 0.08%, particularly suitable for the treatment of ocular conjunctivitis.
- Glycerol 16.5%, water 83.28%, CMCNa 0.12%, *Tanacetum parthenium* extract 0.08%, *Curcuma longa* root extract 0.01%, *Mentha piperita* essential oil 0.01%, particularly suitable for the treatment of pollution induced red eyes and uveitis.
- Glycerol 97.72%, water 1.79%, *Vaccinium macrocarpon* extract 0.16%, *Sambucus nigra* extract 0.33%, citric acid qsp pH 4-5, particularly suitable for the treatment of genital herpes.
- Glycerol 74.56%, water 2.6%, honey 21.97%, xanthan gum 0.16%, *Camellia sinensis* extract 0.21%, *Vitis vinifera* extract 0.32%, *Sambucus nigra* extract 0.18%, particularly suitable for the treatment of labial herpes.
- Glycerol 75.02%, water 3.12%, honey 20.92%, xanthan gum 0.08%, *Vaccinium macrocarpon* extract 0.11%, *Camellia sinensis* extract 0.43%, *Vitis vinifera* extract 0.32%, particularly suitable for the treatment of psoriasis, eczema and dermatitis.
- Glycerol 64.70%, water 34.61%, CMCNa 0.10%, *Vaccinium macrocarpon* extract 0.18%, *Vitis vinifera* extract 0.36%, *Mentha piperita* essential oil 0.05%, particularly suitable for the treatment of external haemorrhoids.
- Glycerol 97.33%, water 1.56%, *Vaccinium macrocarpon* extract 0.23%, *Vitis vinifera* extract 0.36%, *Mentha piperita* essential oil 0.02%, *Linum usitatissimum* oil 0.5%, particularly suitable for the treatment of internal haemorrhoids.
- Glycerol 5.5%, water 83.28%, CMCNa0.12%, *Tanacetum parthenium* extract 0.04%, *Artemisia annua* extract 1.46%, particularly suitable for the treatment of infected ocular injuries.

All finished compositions were stored between 10°C to 40°C in a dark place up to the use. All the products were manufactured in France as per the GMP or ISO13485 norms applicable to medicines or medical devices. All compositions were tested as per the methods and norms proposed in European pharmacopeia and applicable to topically applicable medical products. The compositions were found to be biocompatible with the material of the containers, stable for a period of 3 years, to have in-use stability of 3-months, to be slightly irritant to eyes during the first 1-2 minutes after application, not irritant to skin, not mutagenic or cancerogenic, free of any cellular, metabolic, receptor, or immunologic interactions as they are not absorbed in the body, and to be non-toxic orally up to a dose of 5mL/kg orally and found to have no adverse effects on haematological, blood biochemical, urinary, organ weight, or intracellular histological parameters.

Any multi-factorial topical disease involving cellular destruction, chronic inflammation, and systemic immune stimulation, may produce CRS. As CRS is today incurable, it is difficult to conduct clinical trials by triggering CRS. Therefore, the clinical trials were performed for those diseases involving cellular destruction, chronic inflammation, and systemic immune stimulation susceptible to generate CRS if left untreated.

The clinical efficacy of compositions comprising glycerol and polymer(s) that are only able to bind to glycerol has been compared with the clinical efficacy of compositions comprising glycerol and the dual acting polymer(s) according to the invention.

Comparative clinical efficacy and safety studies were performed in patients having different multifactorial topical pathologies. In general, patients were divided in 2 groups. The first group was treated with placebo "filmogen glycerol" (FG) containing only glycerol binding polymers while the second group was treated with a composition containing the same concentration of dual acting natural and/or synthetic polymers capable of binding with selected disease specific cytokines, proteins or metal ions (CPRMs) and glycerol (DFG). Better efficacy of dual acting polymer(s) containing compositions compared to the corresponding FG composition will reflect additional suppressing of disease related CPRMs to avoid occurrence of CRS.

The dual acting polymer(s) containing compositions were selected among the 35 compositions presented above. The compositions of corresponding FG were identical except that the polymers were replaced by the same concentration of 1, 2, or 3 plant polymers having no binding properties with any of the CPRMs involved in the disease. The first polymer was replaced by *Rubus fruticosus* fruit extract rich in hydrolysable and condensed tannins, the second (if any) by the extract of *Pinus sylvestris* needle extract, the third with *Mangifera indica* leaves and bark extract, and the synthetic polymer (if any) by powdered HPC.

All clinical trials were conducted after the approval of ethical committees following GCP norms. A summary of the experiment and the results obtained for each composition is given below. * indicate statistical difference compared to corresponding placebo at the same time point p<0.05, T0 indicate observation made just before the start of treatment, M = Males, F = Females; control: compositions comprising glycerol and at least one polymer that is not "dual-acting" named "Filmogen glycerol control" = FG; compositions comprising glycerol and the at least one dual acting polymer according to the invention named "Dual acting polymer containing filmogen glycerol" = DFG.

### A - Test composition No 1: (Antiviral & anti-bacterial against severe sore throat)

Composition No 1: glycerol 74.94%, honey 13.57%, water 10.76%, *Vitis vinifera* extract 0.44%, *Sambucus nigra* extract 0.29%. This composition was filled in 30 mL aluminium containers with a spray, particularly suitable for the treatment of throat infection.

Target pathology: Severe sore throat; Type of study: Double blind, placebo controlled, multicentric; Duration of treatment: 14-days; Mode of application: 4-5 sprays on the throat surface, 3-4 times per day; Number of patients: Group 1 (FG) = 20; Group 2 (DFG) = 30.

Parameters: Effect on throat inflammation, pain, difficulty in swallowing, swollen throat, throat irritation, bacterial deposits on the throat surface, and side-effects, if any.

Results: Throat inflammation - The mean score of throat inflammation was not changed in the FG group during the first 24h but a very slight and progressive reduction was observed up to Day 14. The mean scores were 4.58 and 2.47 on days 4 and 7 (5.36/10 at the start) in the FG group versus 1.83* and 0.22* in the DFG group.

When compared with the mean scores of the FG group, the reduction was 20-40*-60*-80* and 90*% superior in the DFG group on Days 2-3-4-7-and 14, respectively. These results clearly show a marked beneficial effect of DFG on throat inflammation, indicating a better cytokine and virus inhibition.

Throat pain: 1h after first application, mean throat pain score decreased by 4.52% in DFG v/s FG. The reduction was faster from Day 2 with a strong reduction between days 3 and 4. On day 7, the mean pain score was reduced by 75% in DFG v/s 28% in the FG group. On day 14, the pain intensity decreased by 55% in FG v/s 93% in the DFG group.

Similar reductions were observed for all other parameters, where recovery was twice faster and complete in the DFG. No side effects were recorded in any patients. These results prove that the DFG composition is more active compared to FG alone.

### B - Test composition No 2 (Antiviral composition for the treatment of influenza infection with severe inflammation, nasal mucosa destruction, and bacterial contamination)

Composition No 2: glycerol 74.94%, water 16.76%, honey 7.57%, CMCNa 0.30%, *Sambucus nigra* extract 0.43%. This composition was filled in 30 mL aluminium containers with a spray, particularly suitable for the treatment of throat infection.

Double blind, placebo controlled, multicentric, pilot study to assess the efficacy and safety of the composition No 2 v/s FG for the treatment of severe throat inflammation and bacterial growth by quantifying the concentrations of inflammatory cytokines, virus particles and bacterial load in throat swabs.

Duration of treatment: 14-days; Mode of application: 4-5 sprays on the throat surface, 3-4 times per day; Number of patients: Group 1 (FG) = 21; Group 2 (DFG) = 40.

Parameters: Throat swabs were collected on day 0 (before treatment), Day 0, 1, 3, and 7. At each time point, one swab was used to quantify virus by introducing the swab in 5 mL cell culture medium, followed by liquid filtration through 0.22 µm filter, and infecting MDCK influenza virus sensitive cell cultures to determine virus quantity in the swab. The second swab was used to quantify bacterial concentration and the third to quantity virus infection specific cytokines (TNF-alpha, IL-4 and IL-6).

Results: The following results were observed on day 1, 3 and 7 (24h after infection):
Mean cell death in control cultures on day 0 - after 24h = 92.2% (FG and DFG groups).

Virus growth induced cell death in FG on days 1, 3, and 7: 78.6%, 71.2%, & 68.4% while in DFG it was 52.3%, 26.1%, and 11.3%.
% patients with 103/mL in swabs bacterial count on day 0 (24h): Mean control = 66.5%

On days 1 (48h), 3, and 7: FG group 29.7%, 21.5%, and 19.6% while in DFG group it was 16.1%, 6.4%, and 0.0%.

Cytokine quantification by ELISA pg/mL, mean concentration before treatment: 811 pg/mL. On days 1, 3, and 7: FG group forTNF-alpha: 656 (±78), 542 (±44), and 523 (±46) pg/mL, while in the DFG group it was 366 (±48), 123 (±36), and 28 (±9) pg/mL.

The IL-4 concentration at the start (day 0 infection, reading 24h) was 89.5 pg/mL. On days 1 (48h), 3 and 7 in the FG group it was 94 (±13), 79 (±14), and 71 (±15) pg/mL, while in the DFG group it was 40 (±9), 11 (±6), and 2.5 (±0.5) pg/mL.

The IL-6 concentration at the start (day 0 infection, reading 24h) was 66.3 pg/mL. On the day 1 (48h), 3, and 7 in FG group was: 55.2 (±7.2), 44.2 (±6.3), and 48.9 (±5.5) pg/mL, while in the DFG group it was 12.3 (±3.1), 2.6 (±0.7), and 0.0 (±0) pg/mL.

All other symptoms of influenza including fever, throat pain, throat tingling and itching were reduced by on average 72% in DFG group compared to the 24% in the FG group within 3 days of treatment. No adverse effects were noticed in any of the patients.

These results clearly prove that dual acting specific disease protein neutralizing DFG composition acts simultaneously on multiple factors and helps faster recovery. Very fast recovery in DFG group is considered to be due to reduction in virus concentration, nasal mucosa healing, and strong decrease in TNF-alpha, IL-4, and IL-6 cytokines involved in the nasal, sinusoidal, upper and lower respiratory tract CRS triggering factors.

### C - Test composition No 3 for the treatment of psoriasis, eczema, & dermatitis

Composition No 3: glycerol 75.02%, water 3.12%, honey 20.92%, xanthan gum 0.08%, *Vaccinium macrocarpon* extract 0.11%, *Camellia sinensis* extract 0.43%, *Vitis vinifera* extract 0.32%. This composition was filled in 50 mL plastic tubes, particularly suitable for the treatment Type of study: Comparative 6-week, single blind clinical trial with composition No 3 to treat Psoriasis, Eczema or Dermatitis (PED) with FG versus DFG for a period of 42 days.

Test product composition No 3, 50 mL tubes containing viscous liquid (FG or DFG).

Number of patients: Group 1 (FG) = 51 (29M +22F); Group 2: (DFG) = 56 (33M + 23F).

Mode of application: Directly on the lesions, topical, 3 times /day for 6 weeks.

Results: Compared to the start of treatment (T0), FG group showed about 15% mean reduction in erythema, pruritis, oedema, oozing, lesion dryness and lesion scaling between week 2 to 6 compared to mean 72% decrease of the same parameters in the DFG group. Statistical significance DFG v/s FG = *. No adverse effects in any of the group.

Conclusion: DFG significantly reduces the signs of erythema & pruritis, lesion oedema, lesion oozing, lesion dryness, itching, scaling, crust formation and improves the quality of life parameters as assessed by the investigators and the patients, right from the second week after the start of treatment. These results show that FG which simply protects, cleans and hydrates the lesions has no effect on cytokine induced cell growth while DFG which acts on multiple disease factors (blocking EGF and FGF induced uncontrolled cell growth) is nearly 4-5 time more effective in treating the disease symptoms.

### D - Test composition No 4 for the treatment of viral rhinosinusitis and respiratory distress in adults

Composition No 4: glycerol 26%, water 73.02%, HPC 0.5%, *Vaccinium macrocarpon* extract 0.07%, *Camellia sinensis* extract 0.13%, *Vaccinium myrtillus* extract 0.07%, SPA 0.21%. This composition was filled in 15 mL plastic containers with a spray, particularly suitable for the treatment of nasal infection (rhinosinusitis).

Type of study: 21-day, single blind, randomized with FG vs DFG.

Test product composition No 4.

Number of patients: Group 1 (FG) = 45; Group 2 (DFG): = 58. Sinusitis severity score (SSS) >10 for the patients included in the trial.

Mode of application: 3-4 sprays per application, twice a day for a maximum period of 21 days. Parameters studied: SSS, need for antibiotic therapy, development of severe respiratory symptoms indicative of CRS.

Results: Although FG sprays proved beneficial after one week of treatment (mean symptom reduction -38%), the DFG treatment produced a much greater, statistically significant, improvement than that of FG with regard to speed and degree of symptom reduction, leading to a lesser need for antibiotherapy.

Additional DFG efficacy versus FG efficacy on days 1, 3, 7, 14, and 21 for (1) nasal congestion was -30.55%, -49.54%, -64.13%, -75.14%, & -87.09%; (2) runny nose was +58.25%, +25.76%, -65.39%, -48.78%, and -52.25%; (3) sinus pain was -62.81%, -76.02%, -66.72%, -48.97%, and -55.63%; (4) overall symptom decrease was -62.1%, -73.09%, -77.46%, -70.05%, and -83.21%.

Six of the 45 patients in the FG group developed severe respiratory symptoms between days 9 to 21 and required hospitalisation versus no patient in the DFG group. Twenty-two of the 45 patients in the FG group required antibiotic therapy compared to 2 of the 58 patients in the DFG group. Statistical significance DFG vs FG = * from day 3. No adverse drug related effects in any of the group.

Conclusion: Viral sinusitis involves presence of virus particles and pro-inflammatory cytokines on the nasal mucosa, nasal mucosa damage, and systemic inflammatory cascade which may lead to severe respiratory distress and triggering of CRS. A composition according to the invention has shown to be effective in reducing rhinosinusitis symptoms compared to FG alone, without any undesired effects, and thus supresses those factors which may trigger CRS.

### E - Test composition No 5 for the treatment of nasal inflammation with infection and respiratory distress

Composition No 5: glycerol 9.8%, water 89.317%, Solagum 0.5%, *Ribes nigrum* extract 0.05%, *Curcuma longa* extract 0.08%, *Mentha piperita* essential oil 0.05%, *Eucalyptus globulus* essential oil 0.05%, *Rosmarinus officinalis* essential oil 0.05%, *Thymus satureioides* essential oil 0.05%, preservatives 0.053% (potassium sorbate 0.025%, sodium benzoate 0.025%, citric acid 0.003%). This composition was filled in 15 mL plastic containers with a spray, particularly suitable for the treatment of nasal infection (rhino-sinusitis)

Type of study: 14-day, comparative, randomized, double blind, parallel group.

Test product: composition No 5.

Patients: Group 1 (FG) = 16; Group 2 (DFG) = 38. Sinusitis severity score (SSS) >10. The enrolled patients had severe respiratory distress, strong sinus, and nasal inflammation, and were hospitalized for respiratory care to avoid occurrence of CRS.

Parameters: Change in rhino-sinusitis severity and overall symptom scores, i.e. sum of rhinorrhoea (anterior nasal discharge), postnasal drip, nasal congestion, headache, facial pain/pressure scores from baseline to 30 minutes after the first dose initiation on day 1, day 2, day 3, day 6, day 14 or day of recovery. Need for antibiotic therapy, respiratory assistance, and total sino-nasal outcome (SNOT) scores on a 1 to 10 scale.

Mode of application: 2-3 sprays per application, three times a day for a maximum period of 14 days.

Key parameters: 50% improvement in respiratory symptoms compared to the start of treatment (T0), need for antibiotic therapy, development of CRS like symptoms, concentration of IL-6 in nasal mucus on Day 0 compared to Day 7.

Results: The DFG was much more effective than the FG for symptomatic and nearly complete relief of rhinosinusitis and respiratory symptoms in patient hospitalized for severe upper respiratory tract inflammation, sinusitis, and prone to CRS. The DFG most probably exerted much stronger pressure over sinuses thereby opening and draining the blocked sinus contents during the first 3 days of treatment as most of the clinical signs (particularly the headache and facial pain) were strongly decreased with strong nasal flow during the first 24h. Progressive respiratory distress improvements were seen from the day 1 of treatment and 50% SNOT symptom reduction within 4 days in DFG group patients compared to 9 days in FG group. Nine of the 16 patients (56.25%) in FG group required antibiotic and/or anti-inflammatory therapy compared to only 7/38 (18.42%) in the DFG group. On day 14, respiratory assistance was maintained in 7/16 (43.75%) patients in FG group compared to only 2/58 (3.44%) in the DFG group. IL-6 nasal swab concentration on days 0 (before first treatment) and 7 was 78 and 42 pg/mL (-41.66%) in FG group compared to 79 and 12 pg/mL (-82.61%) in the DFG group. Both products were totally safe and well tolerated by the patients as none of the patients complained about adverse effects nor any of the patients withdrew from the study.

Conclusion: Composition containing dual acting polymers is nearly twice more effective compared to composition (FG) containing only glycerol binding polymers. The compositions of the invention can be used to supress severe nasal inflammation and to avoid occurrence of CRS.

### F - Test composition No 6 for the treatment of severe hav fever induced allergic rhinitis in adults

Composition No 6: glycerol 2%, water 96.9%, Solagum 0.5%, *Camellia sinensis* extract 0.09%, *Curcuma longa* extract 0.08%, *Panax ginseng* extract 0.06%, *Urtica dioica* extract 0.1%, *Citrus limonum* essential oil 0.05%, preservatives 0.22% (potassium sorbate 0.1%, sodium benzoate 0.1%, citric acid 0.02%). This composition was filled in 15 mL plastic containers with a spray, particularly suitable for the treatment of allergic rhinitis.

A double blind, randomized, placebo controlled, multicentre clinical trial was performed to evaluate the efficacy and safety of FG against DFG for the treatment of allergic rhinitis.

This trial was conducted in patients suffering from severe allergic rhinitis (AR). Thirty-one patients were treated with DFG containing anti-histamine, anti-IgE, anti-IL-2, 4, 6, and 23 dual acting polymers, and 15 patients were treated with FG as comparator product (CP). Both products (15 mL sprays) were applied topically over the nasal mucosa, 3-4 times a day over a period of 3 weeks. Total, reflective and instantaneous nasal symptom scores for rhinorrhoea, nasal discharge, sneezing, and itching, as well as ocular scores (itching, tearing, redness) and rescue medicine use scores were evaluated daily during weeks -1 to +3 employing a 0 (no symptoms) to 3 (severe symptoms) scoring scale. Rhino-conjunctivitis quality of life (RQLQ) questionnaires were completed at the start and at the end of the study. FG was used identically to the DFG. Mean weekly results in both groups were compared with the scores at the start of treatment (baseline) and between the two groups.

Results: FG was found to reduce only slightly the symptomatic manifestation of allergic rhinitis. The mean reduction compared to baseline (T0) at the end of weeks 1, 2, and 3 was respectively 11.7%, 13.6% and 15.1% for total nasal symptom scores (rTNSS); 9.9%, 14.5%, and 15.8% for total ocular symptom scores (rTOSS); and 4.97%, 8.45%, and 10.94% for pre-dose instantaneous total ocular symptom scores (am-iTOSS, p: Not Significant: NS).

During the same period, compared to FG scores, the reduction in DFG group was higher by 37.7%, 58.4%, and 73.5% for rTNSS; 38.3%, 54.6%, and 64.1% for rTOSS and 29.84%, 48.91%, and 59.77% for am-iTOSS (*p<0.05 for all parameters v/s FG at the same time points). The rhino-conjunctivitis quality of life questionnaire (RQLQ), measured using standard established questionnaire, was improved by 50.28% in DFG group compared to 22.85% in FG group. During the study period, at least one rescue medicine was used by 80% patients in FG group compared to only 29% in DFG group. Both products were well tolerated and induced no undesired effects. Conclusion: In the absence of any cell-friendly, safe, and multi-target treatment for allergic rhinitis, using a mechanically acting, filmogen osmotic barrier solution containing specific CPRM inhibiting polymers in filmogen glycerol (DFG) is a highly efficient and safe approach for blocking new allergen contact, cleaning the nasal mucosa of contaminants, neutralizing histamine and IgE, and for stopping inflammatory cascade to reduce the occurrence of CRS.

### G - Test composition No 7 for the treatment of chemotherapy and radiotherapy induced oral mucositis (severe oral and/or gastric inflammation with extensive tissue damage)

Composition No 7: glycerol 73.85%, water 11.94%, honey 13.6%, *Vaccinium macrocarpon* extract 0.28%, *Vaccinium myrtillus* extract 0.33%. This composition was filled in 20 mL aluminium containers with a spray, particularly suitable for the treatment of chemotherapy induced oral mucositis.

A single blind, randomized, placebo controlled clinical trial to evaluate the efficacy and safety of DFG against commonly used anti-inflammatory and/or antibiotic drugs for the treatment of radiotherapy and chemotherapy induced oral mucositis involving severe oral and gastric inflammation and may lead to death because of CRS. Sixty-nine patients were included in the study. Forty-eight patients were treated with DFG and on 21 with commonly used oral topical drugs as control group.

Oral mucositis ulcers were treated 4-5 times per day for a period of 28 days. The grade of overall mucositis, intensity of pain and burning sensation, formation of new ulcers and effect on eating impairment, were evaluated before treatment, 30 minutes after first product application and on days 1, 2, 3, 4, 7, 14, 21 and 28. Symptoms were scored on a scale of 0 (no symptoms) to 4 (severe symptoms) or 0 to 10 for certain parameters.

Results: compared to the commonly used drug treatment group, DFG group showed significantly higher improvement on day 28 for oral mucositis (0.83/4 in DFG vs 2.10/4 for controls), pain (2.05/10 vs 5.71/10 controls), oral and gastric burning sensation (1.92/10 vs 5.76 for controls), eating abilities, infection of the lesions (0.52/4 vs 1.95 for controls), and for the improvement to take solid food (2.38/4 vs 1.50/4 for the control group). New ulcer formation rate was not affected. As mucositis treatment requires a multiple therapeutic approach of simultaneously eliminating the contaminants and the toxic chemicals from the ulcer as well as creating a favourable ground for healthy cell growth, DFG was at least 3-4 times more effective in achieving these objectives compared to standard anti-inflammatory or anti-bacterial drugs.

### H - Composition No 8 for the treatment of severe chronic cough in adults

Composition No 8: glycerol 40%, water 50.43%, honey 8%, *Calendula officinalis* extract 0.5%, *Eucalyptus globulus* essential oil 0.02%, *Linum usitatissimum* oil 0.05%, propolis extract 1%. This composition was filled in 30 mL aluminium containers with a spray, particularly suitable for the treatment of dry cough.

Type of study: A 14-day, randomized, placebo-controlled, double blind, efficacy and safety study was conducted by applying the cough spray (30 mL spray), 4-5 sprays, 3-4 times per day for 14 consecutive days.

Number of patients: 17 in the FG group v/s 37 in DFG group.

Parameters: The primary outcome was defined as effects on dry cough severity and cough frequency, as well as changes in throat irritation, throat pain, effect on throat inflammation (swelling, throat redness) and related parameters, which are summarized in Leicester Cough Questionnaire (LCQ) for cough-related quality of life. Changes were evaluated on a rating scale of 0 to 10 (0 indicating absence of symptoms) just before 1st treatment, after 5 min and 2 h, and on day 1, 2, 3, 6, 9 and 14 by the investigator (day 0, 1, and 14) or by the patients.

Results: Compared to FG treatment, the DFG treatment triggered an instant and strong reduction in the mean scores of dry cough severity (ex. day 9: mean score was 5.8/10 in FG vs 3.6/10 for DFG), cough frequency (day 9: 5.3/10 in FG vs 2.2/10 in DFG), throat irritation (4.8/10 in FG vs 1.6/10 in DFG), throat inflammation (4.7/10 in FG vs 0.8/10 in DFG, with remarkable improvement in Leicester cough questionnaire parameters. The DFG was at least 2 to 3 times more rapid and effective in relieving cough symptoms compared to FG treatment alone. DFG strongly reduces inflammation and microbial contamination with a long-lasting mucus fluidizing property. No cases of CRS were observed in any of the patients after treatment termination.

### I - Composition No 9 for the treatment of throat inflammation & sore throat in children

Composition No 9: glycerol 74.63%, honey 6%, water 18.54%, *Vitis vinifera* extract 0.44%, *Sambucus nigra* extract 0.29%, *Ribes nigrum* extract 0.1%. This composition was filled in 20 mL aluminium containers with a spray, particularly suitable for the treatment of throat infection and pain in children.

Type of study: Clinical efficacy and safety of composition No 9 directed for the treatment of inflammatory sore throat in children between 3-15 years.

Product presentation: both products in 20 mL aluminium containers with a spray. Number of patients: Group 1 (FG) = 9; Group 2 (DFG) = 20. Mode of application: 3-4 sprays on the throat surface per application, 4-5 times a day for a maximum period of 14 days. Parameters: effect on throat inflammatory parameters on day 1, day 3, day 6, day 14-15 or day of recovery (whichever earlier).

Results: % improvement (+) or worsening (-) of clinical symptoms in DFG versus FG group on days 1, 2, 6, and 14 for: (1) difficulty in swallowing +11.48%; +42.59%, +74.42%, and +93.33%; (2) swollen throat +5.38%, +30.99%, +52.94%, and +91.43%; (3) throat irritation +2.08%, +22.37%, +60.34%, and +97.18%; (4) throat inflammation +5.0%, +40.1%, +72.58 and +97.37%; and (5) bacterial deposit on throat surface: +2.27% (+2h), +41.43 on day 3, +59.09% on day 6, and +88.89% on day 14.

Presence of microorganisms in throat swabs: At the start of the study, 8/9 children in the FG group and 15/20 in the DFG group had a positive throat swab for the presence of bacteria. On day 6, only 2/9 children in the FG group and 3/20 in the DFG group were positive. These results prove a much stronger and rapid effect of DFG versus FG alone to suppress throat inflammation in children without any additional adverse effect. An excellent anti-inflammatory effect was observed right after 2-days of treatment with DFG composition.

### J - Composition No 10 for the treatment of dry cough in children

Composition No 10: glycerol 53%, water 41.81%, honey 4.5%, *Ribes nigrum* extract 0.36%, *Curcuma longa* extract 0.08%, *Citrus limonum* essential oil 0.02%, preservatives 0.23% (potassium sorbate 0.1%, sodium benzoate 0.1%, citric acid 0.03%). This composition was filled in 20 mL aluminium containers with a spray, particularly suitable for the treatment of cough in children.

Type of study: Clinical efficacy and safety of two dry cough treatment compositions: DFG against FG, for the treatment of dry cough in children. Number of patients: Group 1 (FG) = 9; Group 2 (DFG) =20 (age between 3-15 years). Mode of application: both products were presented in 15 mL sprays and were applied as 3-4 sprays on the throat surface per application, 4-5 times a day for a maximum period of 15 days. Parameters: severity and frequency of cough, chest discomfort, and throat irritation at 2 h after the first application on day 1, day 3, day 6 and day 15 or up to complete recovery.

Results: % change DFG versus FG composition after 2h, and days 3, 6, and 15 for: (1) coughing: 0.0%, -45.79%, -89.71%, and -99.0%; (2) chest discomfort: -3.19%; -47.72%, -75.48%, and - 91.66%; (3) throat irritation: 0.0%, -66.6%, -88.2%, and -98.0%; (4) quality of life (QOL): the mean score of quality of life parameters was nearly equal in both the groups at the beginning of the study. After 14 days of treatment, the QOL was highly improved in the DFG group (mean score 41.60) compared to the FG group (23.30); and (5) need for antibiotic therapy: 70% in the FG group compared to 25% in the DFG group.

Conclusion: DFG composition is nearly twice more effective in reducing dry cough symptoms in children compared to the FG alone treatment.

### K - Composition No 11 for the treatment of chronic inflammatory Rhinosinusitis in children

Composition No 11: glycerol 13%, water 85.96%, HPC 0.5%, *Vaccinium macrocarpon* extract 0.07%, *Vaccinium myrtillus* extract 0.07%, *Sambucus nigra* extract 0.05%, *Ribes nigrum* extract 0.13%, preservatives 0.22% (potassium sorbate 0.1%, sodium benzoate 0.1%, citric acid 0.02%). This composition was filled in 15 mL plastic containers with a spray, particularly suitable for the treatment of nasal infection in children.

Type of study: A 14-day comparative, randomized, double-blind, parallel group, observational clinical trial to evaluate chronic inflammation supressing efficacy of FG versus DFG in children suffering from chronic rhinosinusitis.

Test product presentation and use: 15 mL nasal sprays, 2-3 sprays per application, twice a day for a maximum period of 14 days. Number of patients: Group 1 (FG) = 10; Group 2 (DFG) =20. Parameters: Rhino-sinusitis Severity Score (RSSS) at 30 minutes after the first dose initiation on day 1, day 3, day 6, day 14-15 or day of recovery (whichever earlier), effect on rhinorrhoea or nasal congestion, fever, cough, lack of good sleep, pain upon facial pressure.

Results: % improvement (+) or worsening (-) of clinical symptoms with Dual acting FG efficacy v/s FG efficacy on days 1, 2, 6, and 14 for: (1) rhinorrhoea: +11.69%; +44.12%, +61.70%, and +95.0% ; (2) coughing: -2.13%, +35.65%, +52.81%, and +93.62%; (3) effect on sleep: 0.0%, +50.40%, +62.89%, and +97.18%; (4) effect on facial pain upon pressure: +2.27%, +41.51%, +57.89%, and +93.31%; (5) effect on fever: 57% reduction in the fever score in the DFG group vs FG group; and (6) product efficacy: 90% patients in DFG group rated the product efficacy as excellent vs 15% in FG group (65% patients in FG group rated product as fair or good).

Conclusion: DFG composition was considered highly effective in suppressing the symptoms of chronic inflammatory rhinosinusitis in children compared to the FG composition. Both compositions were well tolerated by children. The reduction of clinical signs indicates a strong anti-inflammatory effect of DFG which could be related to the suppression of pro-inflammatory cytokines.

### L - Composition No 12 for the treatment of ocular and nasal inflammatory allergic rhinitis in children

Composition No 12: glycerol 4%, water 94.87%, Solagum 0.6%, *Vaccinium macrocarpon* extract 0.1%, PVOH 0.1%, *Panax ginseng* extract 0.06%, *Thymus satureioides* essential oil 0.03%, *Citrus limonum* essential oil 0.02%, preservatives 0.22% (potassium sorbate 0.1%, sodium benzoate 0.1%, citric acid 0.02%). This composition was filled in 15 mL plastic containers with a spray, particularly suitable for the treatment of allergic rhinitis in children.

Type of study: 14-day, single blind, randomized with FG versus DFG composition. Number of patients: FG group = 10; Group 2: DFG group = 20. Allergen induced sinusitis severity score (SSS) >10. Mode of application: 15 mL sprays were supplied and applied as 3-4 sprays per application, twice a day for a maximum period of 14 days in both groups.

Parameters: runny nose, TNSS (Total Nasal Symptom Score), eye redness, TOSS (Total Ocular Symptom Score). Histamine concentration was determined in nasal swabs collected on day 0 (before treatment) and on day 1 (24h) in 8 random patients of each group. The swabs were washed with 1 mL saline, and the histamine concentration was measured using histamine ELISA kit (ABCAM-ab213975) at 450 nm.

Results: Although FG sprays proved beneficial, the DFG produced a much greater, statistically significant, improvement regarding speed and degree of symptom reduction, leading to a lesser need for anti-inflammatory therapy.

Improved DFG efficacy versus FG efficacy on days 1, 2, 7, and 14 for: (1) runny nose: +12.68%; - 28.17%, -73.24%, and -91.55%; (2) TNSS: -2.59%, -18.75%, -56.66%, and -87.50%; (3) eye watering: 0.0%, -18.57%, -55.71%, and -100.0%; (4) eye itching: -5.75%, -41.38%, -89.66%, and - 98.85%; (5) eye swelling: -9.09%, -34.64%, -77.27%, and -100.0%; (6) TOSS: -5.30%, -13.73%, - 75.60%, and -100.0%;(7) Histamine at T0 and T24h: the mean concentrations detected in FG group were 8.35 and 3.60 nmol versus 7.80 and 0.20 in the DFG group, indicating a strong topical histamine blocking activity of DGF composition compared to FG formulation; and (8) QOL on day 7: 52.7% in FG versus 72.61 in DFG group.

These results show that the DFG test product was much more effective in reducing the symptomatic manifestation of allergic rhinitis in children compared to the FG alone due to its specific histamine and pro-inflammatory cytokine blocking properties. The beneficial effects are observed after 3-7 days of treatment. DFG formulations were much better and faster. The mode of action of this effect may be related to the higher CPRM binding properties of DFG formulation compared to FG.

### Conclusion on pharmacological and clinical data

These results prove that the clinical efficacy of compositions comprising dual acting polymer(s) and glycerol (DFG) is much superior compared to the filmogen glycerol (FG) for the treatment of all topical inflammatory diseases. Why and when CRS may occur is not yet understood but it has been observed in all kind of chronic inflammatory diseases. The intensity, severity and consequences of CRS vary according to the site and the type of disease. CRS affecting vital organs such as the lungs or the heart, are detected easily as they may cause respiratory or cardiac failure. The chronic topical disease physiopathology usually involves the continuation of pathogenic insult, extensive cellular damage, topical and systemic presence of excessively high concentrations of multiple disease related pro-inflammatory cytokines and proteins, which maintains chronic inflammatory cascade. The CRS is the consequence of a chain of reactions where each factor is inter-dependent on the other and all these factors are already activated when clinical signs of the disease appear. Therefore, any effective treatment must be multi-target to block partially or totally all the causes involved in the triggering of CRS.

The results of these topical compositions containing dual acting polymers with osmotic glycerol prove that these compositions can be used safely to supress multiple factors involved in the triggering of CRS.

### REFERENCES

Dang Xuan Cuong et al. Tannin extraction from plants. Chapter in book - Structural properties, Biological properties and current knowledge: Chapter on Tannin extraction. IntechOpen publisher, May 13th 2019. doi: 10.5772/intechopen.86040.
Maria Fraga-Corral et al. Technological application of tannin-based extracts. Molecules 2020, 25(3)614. doi: 10.3390/molecules25030614.

## Claims

1. A composition comprising at least one dual acting polymer bound to glycerol and able to bind to at least one pro-inflammatory compound, for topical use in the prevention or the treatment of topical inflammatory disease and of the cytokine release syndrome, in a subject in need thereof.

2. The composition according to claim 1, wherein the at least one pro-inflammatory compound is selected from the group consisting of matrix metalloproteases, histamines, cytokines, cellular receptors, metal ions, immunoglobulins or viral glycoproteins.

3. The composition according to any one of the preceding claims, wherein the topical inflammatory disease is selected from the group consisting of viral infections, rhinosinusitis, wounds and ulcers, psoriasis, eczema, dermatitis, allergy, asthma, pollution induced respiratory diseases, pollution induced topical damage, gastro-intestinal ulcers, haemorrhoids, genital infections, ocular allergy, conjunctivitis, and ocular inflammation.

4. The composition according to any one of the preceding claims, wherein the total amount of the at least one dual acting polymer is ranging from 0.01% to 5% by weight of total weight of said composition, preferably from 0.01% to 3.5% by weight of total weight of said composition.

5. The composition according to any one of the preceding claims, further comprising at least one ingredient selected from the group consisting of honey, propolis extract, vegetable gum such as xanthan gum and/or acacia gum, and essential oils.

6. The composition according to any one of the preceding claims, further comprising at least one medication, such as an analgesic, antibiotic, anti-inflammatory drug, antihistamine, vasodilator, bronchodilator, antioedematous drug, specific topical receptor binding compound or an essential oil.

7. The composition according to any one of the preceding claims, wherein said composition is topically applied on a damaged and/or inflammatory biological surface such as the skin, eye mucosa, conjunctiva, cornea, oral, nasal, gastrointestinal, respiratory, or genital surfaces.

8. The composition according to any one of the preceding claims, wherein said composition is administered as a liquid, inhaler, liquid bandage, solution, gel, cream, paste, or ointment, presented in sprays, tubes, ampoules, liquid embedded cotton or polymeric bandages, granules, powder or soft-gel capsules.

9. The composition according to any one of the preceding claims, wherein the at least one dual acting polymer is natural, semi-synthetic and/or synthetic.

10. The composition according to claim 9, wherein the at least one dual acting polymer is a tannin obtained from a plant or parts of the plant.

11. The composition according to claims 5 or 6, wherein the ingredient and/or the medication is captured into the polymeric linkages of said composition, for sustained release of the ingredient and/or of the medication, to further enhance therapeutic properties of the composition.

## Patentansprüche

1. Zusammensetzung umfassend mindestens ein doppelt wirkendes Polymer, das an Glycerin gebunden und in der Lage ist, an mindestens eine proinflammatorische Verbindung zu binden, zur topischen Anwendung bei der Vorbeugung oder Behandlung von topischen entzündlichen Erkrankungen und des Zytokinfreisetzungssyndroms bei einem Subjekt, das dies benötigt.

2. Zusammensetzung nach Anspruch 1, wobei die mindestens eine proinflammatorische Verbindung aus der Gruppe ausgewählt ist, die aus Matrixmetalloproteasen, Histaminen, Zytokinen, zellulären Rezeptoren, Metallionen, Immunglobulinen oder viralen Glykoproteinen besteht.

3. Zusammensetzung nach einem der voranstehenden Ansprüche, wobei die topische entzündliche Erkrankung aus der Gruppe ausgewählt wird, die aus Virusinfektionen, Rhinosinusitis, Wunden und Geschwüren, Psoriasis, Ekzemen, Dermatitis, Allergie, Asthma, durch Umweltverschmutzung verursachten Atemwegserkrankungen, durch Umweltverschmutzung verursachten topischen Schäden, Magen-Darm-Geschwüren, Hämorrhoiden, Genitalinfektionen, Augenallergie, Bindehautentzündung und Augenentzündung besteht.

4. Zusammensetzung nach einem der voranstehenden Ansprüche, wobei die Gesamtmenge des mindestens einen doppelt wirkenden Polymers im Bereich von 0,01 % bis 5 % des Gesamtgewichts der Zusammensetzung liegt, vorzugsweise im Bereich von 0,01 % bis 3,5 % des Gesamtgewichts der Zusammensetzung.

5. Zusammensetzung nach einem der voranstehenden Ansprüche, weiterhin umfassend mindestens einen Inhaltsstoff, ausgewählt aus der Gruppe bestehend aus Honig, Propolis-Extrakt, pflanzlichem Gummi wie Xanthan und/oder Akaziengummi, und ätherischen Ölen.

6. Zusammensetzung nach einem der voranstehenden Ansprüche, weiterhin umfassend mindestens ein Medikament, wie z.B. ein Analgetikum, ein Antibiotikum, ein entzündungshemmendes Mittel, ein Antihistaminikum, ein gefäßerweiterndes Mittel, ein bronchienerweiterndes Mittel, ein Mittel gegen Ödeme, eine Verbindung, die einen spezifischen topischen Rezeptor bindet, oder ein ätherisches Öl.

7. Zusammensetzung nach einem der voranstehenden Ansprüche, wobei die Zusammensetzung topisch auf eine geschädigte und/oder entzündete biologische Oberfläche wie die Haut, die Augenschleimhaut, die Bindehaut, die Hornhaut, die oralen, nasalen, gastrointestinalen, respiratorischen oder genitalen Oberflächen aufgetragen wird.

8. Zusammensetzung nach einem der voranstehenden Ansprüche, wobei die Zusammensetzung als Flüssigkeit, Inhalator, Flüssigverband, Lösung, Gel, Creme, Paste oder Salbe verabreicht wird, die in Sprays, Tuben, Ampullen, in Flüssigkeit eingebetteten Baumwoll- oder Polymerbinden, Granulat, Pulver oder Weichgelkapseln dargereicht wird.

9. Zusammensetzung nach einem der voranstehenden Ansprüche, wobei das mindestens eine doppelt wirkende Polymer natürlich, halbsynthetisch und/oder synthetisch ist.

10. Zusammensetzung nach Anspruch 9, wobei das mindestens eine doppelt wirkende Polymer ein Tannin ist, das aus einer Pflanze oder Teilen der Pflanze gewonnen wurde.

11. Zusammensetzung nach Anspruch 5 oder 6, wobei der Inhaltsstoff und/oder das Medikament für eine anhaltende Freisetzung des Inhaltsstoffs und/oder des Medikaments in den polymeren Bindungen der Zusammensetzung eingeschlossen ist, um die therapeutischen Eigenschaften der Zusammensetzung weiter zu verbessern.

## Revendications

1. Composition comprenant au moins un polymère à double action lié au glycérol et apte à se lier à au moins un composé pro-inflammatoire, pour une utilisation topique dans la prévention ou le traitement d'une maladie inflammatoire topique et du syndrome de relargage des cytokines, chez un sujet en ayant besoin.

2. Composition selon la revendication 1, dans laquelle l'au moins un composé pro-inflammatoire est choisi dans le groupe constitué par les métalloprotéases matricielles, histamines, cytokines, récepteurs cellulaires, ions métalliques, immunoglobulines ou glycoprotéines virales.

3. Composition selon l'une quelconque des revendications précédentes, dans laquelle la maladie inflammatoire topique est choisie dans le groupe constitué par les infections virales, la rhinosinusite, les plaies et ulcères, le psoriasis, l'eczéma, la dermatite, l'allergie, l'asthme, les maladies respiratoires induites par la pollution, une lésion topique induite par la pollution, les ulcères gastro-intestinaux, les hémorroïdes, les infections génitales, l'allergie oculaire, la conjonctivite, et l'inflammation oculaire.

4. Composition selon l'une quelconque des revendications précédentes, dans laquelle la quantité totale de l'au moins un polymère à double action est dans la plage de 0,01 % à 5 % en poids du poids total de ladite composition, de préférence de 0,01 % à 3,5 % en poids du poids total de ladite composition.

5. Composition selon l'une quelconque des revendications précédentes, comprenant en outre au moins un ingrédient choisi dans le groupe constitué par le miel, l'extrait de propolis, la gomme végétale telle que la gomme xanthane et/ou la gomme d'acacia, et les huiles essentielles

6. Composition selon l'une quelconque des revendications précédentes, comprenant en outre au moins un médicament, tel qu'un analgésique, antibiotique, médicament anti-inflammatoire, antihistaminique, vasodilatateur, bronchodilatateur, médicament anti-œdémateux, composé de liaison de récepteur optique spécifique ou une huile essentielle.

7. Composition selon l'une quelconque des revendications précédentes, dans laquelle ladite composition est appliquée de manière topique sur une surface biologique endommagée et/ou inflammatoire telle que la peau, muqueuse oculaire, conjonctive, cornée, les surfaces orales, nasales, gastrointestinales, respiratoires, ou génitales.

8. Composition selon l'une quelconque des revendications précédentes, dans laquelle ladite composition est administrée en tant que liquide, inhalateur, bandage liquide, solution, gel, crème, pâte, ou onguent, présentés dans des aérosols, tubes, ampoules, bandages en coton ou polymère incorporés dans un liquide, granules, capsules de poudre ou à enveloppe molle.

9. Composition selon l'une quelconque des revendications précédentes, dans laquelle l'au moins un polymère à double action est naturel, semi-synthétique et/ou synthétique.

10. Composition selon la revendication, dans laquelle l'au moins un polymère à double action est un tanin obtenu à partir d'une plante ou de parties de la plante.

11. Composition selon les revendications 5 ou 6, dans laquelle l'ingrédient et/ou le médicament est capturé dans les liaisons polymères de ladite composition, pour une libération prolongée de l'ingrédient et/ou du médicament, pour continuer d'améliorer les propriétés thérapeutiques de la composition.
